(19) Europäisches Patentamt
European Patent Office
Office européen des brevets

(11) EP 0 255 085 B2

(12) NEW EUROPEAN PATENT SPECIFICATION

(45) Date of publication and mention of the opposition decision:
**07.08.1996 Bulletin 1996/32**

(45) Mention of the grant of the patent:
**06.06.1990 Bulletin 1990/23**

(21) Application number: **87110858.5**

(22) Date of filing: **27.07.1987**

(51) Int Cl.[6]: **G03C 7/32**
// C07D257/04

# (54) Photographic element and process

Photographisches Element und Verfahren

Elément photographique et procédé

(84) Designated Contracting States:
**BE CH DE FR GB IT LI NL**

(30) Priority: **30.07.1986 US 890674**

(43) Date of publication of application:
**03.02.1988 Bulletin 1988/05**

(73) Proprietor: **EASTMAN KODAK COMPANY
(a New Jersey corporation)
Rochester, New York 14650 (US)**

(72) Inventors:
- **Burns, Paul Andrew Eastman Kodak Company
Rochester New York 14650 (US)**
- **Taber, Terry Ray Eastman Kodak Company
Rochester New York 14650 (US)**

(74) Representative: **Brandes, Jürgen, Dr. rer. nat. et al
Wuesthoff & Wuesthoff
Patent- und Rechtsanwälte
Schweigerstrasse 2
81541 München (DE)**

(56) References cited:
**EP-A- 0 089 834**
**DE-A- 3 518 231**
**GB-A- 1 531 927**
**GB-A- 2 072 363**
**US-A- 4 248 962**

- **JP-A-60 218 645 & English translation**
- **JP-A-61 156 127 & English translation**

EP 0 255 085 B2

## Description

This invention relates to new photographic compounds which release photographically useful groups during photographic processing and to photographic materials and processes using such compounds.

Various ways are recognized in the photographic art for release of a photographically useful group (PUG) from a compound, such as a coupler, in photographic materials and processes. For example, U.S. Patent 4.248.962, describes compounds that release a photographically useful group by means of an intramolecular nucleophilic displacement reaction in photographic materials. Other examples are described in U.S. Patent 4.409,323, wherein couplers are described which release a photographically useful group by means of an electron transfer down a conjugated chain. These compounds capable of releasing a photographically useful group in a photographic material upon processing provide a degree of control over the timing and rate of release as well as the rate of diffusion and distance of diffusion of the photographically useful group in the photographic material.

A need has continued to exist for a higher degree of control over these parameters as well as a higher degree of freedom in the capability to design compounds having releasable photographically useful groups. Moreover, such needs have existed with the added parameter that such compounds must not require significantly modifying the photographically useful groups or the carrier compounds, such as the couplers, from which the photographically useful group are released, in a way which would be inconsistent with the ultimate use for which each is intended.

The present invention solves these problems by means of a photographic element comprising a support, at least one photographic emulsion layer and at least one compound A as represented by the structure of the following formula:

$$COUP - (T_1) - (T_2)\text{-}PUG$$

wherein:

COUP is a coupler moiety;

PUG is a photographically useful group and

$T_1$ and $T_2$ are different first and second timing groups, which control the release of PUG, which is bonded to $T_2$; the first timing group $T_1$ is capable of being released from COUP at the coupling position; said compound A forms two different fragments from the two different timing groups $T_1$ and $T_2$ upon processing the photographic element, wherein

> $T_1$ is a group capable of an intramolecular nucleophilic displacement reaction, said group comprising a nucleophilic and an electrophilic group which form a cyclic organic ring or a transient cyclic organic ring by the intramolecular reaction, and
>
> $T_2$ is a group capable of electron transfer down a conjugated chain.

Wherever in the following reference is made to a timing group either $T_1$ or $T_2$ the above given definitions apply.

The compound A contains two differing timing groups, in sequence capable upon activation of sequentially timing the release of a PUG. The reaction of compound A

with oxidized color developing agent cleaves the bond between the first timing group ($T_1$) and the carrier portion of compound A, preferably the coupler moiety (COUP). Then the bond between the first timing group ($T_1$) and the second timing group ($T_2$) is cleaved. Finally, the bond between the second timing group ($T_2$) and the PUG is cleaved enabling the PUG to perform its intended function. Bond cleavage between $T_1$ and $T_2$ or between $T_2$ and PUG preferably does not involve the action of oxidized color developer. The sequential cleavage of the bond between the carrier portion of compound A and the first timing gorup, then the bond between the first timing group ($T_1$) and the second timing group ($T_2$), and finally the bond between the PUG and the second timing group ($T_2$) enables the improved control over timing and rate of release of PUG. The sequential cleavage of these bonds is a characteristic feature of the invention.

One advantage of compounds as described is the greater variety of workable linking groups available for organic synthesis. Another advantage is that for the first time an extended timing period is available during which none of the PUG is released. For a given PUG, available single timing groups may provide a release rate too fast or too slow for the desired application, while two differing timing groups in sequence allows flexibility in attaining the desired release rate, typically with a half-life in the 0.1 to 60 second range, such as 15 seconds to 60 seconds. $T_1$ and $T_2$ are particularly useful when they have essentially matching timing of release.

For compounds, such as couplers, involving release of a development inhibitor group in a photographic element, a coupler as described enables more control over image sharpness, granularity, and balanced color reproduction without deleterious effects on desired properties, such as photographic speed and sensitometric curve shape. For compounds, such as couplers, involving release of a bleach accelerator group in a photographic element, this improved control of timed release enables processing steps, such as color development, prior to bleaching to proceed to completion without interference from prematurely released bleach accelerator. In pnotographic elements, particularly photographic elements involving diffusion of compounds and/or fragments of compounds between layers, the controlled delayed release according to the invention enables larger diffusion paths of a released fragment before release of a PUG and enables improved control of interlayer interimage effects.

A particularly useful coupler is represented by the formula:

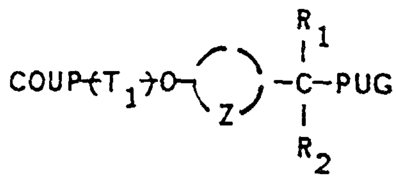

where COUP, $T_1$ and PUG are the same as described while Z represents atoms necessary to complete a substituted or unsubstituted pyridine, pyrazole, benzene or naphthalene nucleus and $R_1$ and $R_2$ individually represent a hydrogen atom, alkyl or aryl, with the group

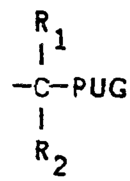

being joined to the nucleus at the para or ortho position relative to the oxygen atom.

In chemical systems requiring timed release of a moiety the release mechanisms can be initiated by any means that initiates cleavage of the first timing group from the carrier moiety. Depending on the particular carrier compound, the particular timing groups, and the desired end use of the active moiety, the release mechanism can be initiated by, for example, reaction of the carrier compound with radiation, enzymes, moisture, acid or base, and/or oxidized reducing agent.

As used herein the terms "coupler" and "coupler compound" refer to the entire compound, including the coupler moiety, the timing groups and the PUG, while the term "coupler moiety" refers to that portion of the compound other than the timing groups and the PUG.

The particular timing groups employed, including the linkage by which they are attached to other portions of coupler and the nature of the substituents on them, can be varied to help control such parameters as rate and time of cleavage of the timing groups and of the PUG. Since these parameters can be controlled by modification of the timing groups, they need not be emphasized in selecting the particular coupler moiety and the particular PUG, thus providing greater freedom in selecting such moieties and groups for a particular end use.

The PUG is joined to the coupler moiety only through the timing groups, wherein cleavage of the bond between the first timing groups and the coupler moiety release the timing groups and the PUG as a unit. The particular timing groups employed, including the nature of the substituents on them, can additionally control the rate and distance of diffusion of the unit formed by the timing groups and the PUG after this unit is released from the coupler moiety but before the PUG is released from the second timing group. If the PUG is joined to the coupler moiety both directly and through the timing groups, the particular timing groups and the nature of the substituents on them can control the rates of cleavage of the timing groups and can control the rate at which the PUG is released. Direct linkage between the PUG and the coupler moiety helps prevent diffusion of the PUG.

The coupler moiety can be any moiety which will react with oxidized color developing agent to cleave the bond between the first timing group and the coupler moiety. It includes coupler moieties employed in conventional color-forming couplers which yield colorless products on reaction with oxidized color developing agents as well as coupler moieties which yield colored products on reaction with oxidized color developing agents. Both types of coupler moieties are well known to those skilled in the art.

The coupler moiety can be unballasted or ballasted with an oil-soluble or fat-tail group. It can be monomeric, or it can form part of a dimeric, oligomeric or polymeric coupler, in which case more than one COUP $\{T_1\}\{T_2\}$PUG group can be contained in the coupler, or it can form part of a bis compound in which the $T_1$, $T_2$ and/or PUG groups form part of the link between two coupler moieties.

It will be appreciated that, depending upon the particular coupler moiety, the particular color developing agent and the type of processing, the reaction product of the coupler moiety and oxidized color developing agent can be: (1) colored and nondiffusible, in which case it will remain in the location where it is formed: (2) colored and diffusible, in which case it may be removed during processing from the location where it is formed or allowed to migrate to a different location or (3) colorless and diffusible or nondiffusible. in which case it will not contribute to image density. In cases (2) and (3) the reaction product may be initially colored and/or nondiffusible but converted to colorless and/or diffusible

products during the course of processing.

The $-(T_1)-(T_2)-PUG$ group is joined to the coupler moiety at any of the positions from which groups released from couplers by reaction with oxidized color developing agent can be attached. The $-(T_1)-(T_2)-PUG$ group is attached at the coupling position of the coupler moiety so that upon reaction of the coupler with oxidized color developing agent the $-(T_1)-(T_2)-PUG$ group will be displaced. However, the $-(T_1)-(T_2)-PUG$ group can be in a non-coupling position of the coupler moiety from which position it will be displaced as a result of reaction of the coupler with oxidized color developing agent. Alternatively, the coupler moiety can have a $-(T_1)-(T_2)-PUG$ group in each of the coupling position and a non-coupling position. Accordingly, the couplers can release more than one mole of PUG per mole of coupler. The PUGs can be the same or different and can be released at the same or different times and rates.

The first timing group ($T_1$) serves to connect COUP to the second timing group ($T_2$) and which, after cleavage from COUP will cleave from the second timing group ($T_2$), preferably by an intramolecular nucleophilic displacement reaction of the type described in, for example, U.S. Patent 4,248.962.

As used herein, the term "intramolecular nucleophilic displacement reaction" refers to a reaction in which a nucleophilic center of a compound reacts directly, or indirectly through an intervening molecule, at another site on the compound, which is an electrophilic center, to effect displacement of a group or atom attached to the electrophilic center. Such compounds have a nucleophilic group and electrophilic group spatially related by the configuration of the molecule to promote reactive proximity. Preferably the nucleophilic group and the electrophilic group are located in the compound so that a cyclic organic ring, or a transient cyclic organic ring, can be easily formed by an intramolecular reaction involving the nucleophilic center and the electrophilic center.

A nucleophilic group is understood to be a grouping of atoms one of which is electron rich. This atom is referred to as the nucleophilic center. An electrophilic group is understood to be a grouping of atoms one of which is electron deficient. This atom is referred to as the electrophilic center.

Thus, in the photographic couplers as described, the first timing group preferably contains a nucleophilic group and an electrophilic group which are spatially related with respect to one another so that upon release from the coupler moiety the nucleophilic center and the electrophilic center will react to effect displacement of the second timing group and PUG from the first timing group. In order to assure that the second timing group and the PUG are not released prior to release of the first timing group from the coupler moiety, the nucleophilic center should be prevented from reacting with the electrophilic center until such release and the electrophilic center should be resistant to external attack, e.g. hydrolysis. Premature reaction can be prevented by attaching the coupler moiety to the first timing group at the nucleophilic center or an atom in conjunction with a nucleophilic center, so that cleavage of the timing group and PUG from the coupler moiety unblocks the nucleophilic center and permits it to react with the electrophilic center, or by positioning the nucleophilic group and the electrophilic group so that they are prevented from coming into reactive proximity until release. Similarly, the second timing group will be attached at a position on the first timing group from which it will be displaced upon reaction of the nudeophilic center and the electrophilic center.

The second timing group ($T_2$) is an organic group different from the first timing group ($T_1$), which will serve to connect the first timing group ($T_1$) to the PUG, and which, after cleavage from the first timing group ($T_1$), will cleave from the PUG. The cleavage of the second timing group ($T_2$) from the PUG is by means of an electron transfer down a conjugated chain.

As used herein the term "electron transfer down a conjugated chain" is understood to refer to transfer of an electron along a chain of atoms in which alternate single bonds and double bonds occur. A conjugated chain is understood to have the same meaning as commonly used in organic chemistry. Electron transfer down a conjugated chain is as described in, for example, U.S. Patent 4,409,323.

The timing groups ($T_1$ and/or $T_2$) can contain moieties and substituents which will permit control of (i) one or more of the rates of reaction of COUP with oxidized color developing agent, (ii) the rate of diffusion of $-(T_1)-(T_2)-PUG$ and/or $-(T_2)-PUG$ and (iii) the rate of release of PUG. The timing groups can contain additional substituents, such as additional PUGs, or precursors thereof, which may remain attacned to the timing groups or be released.

The PUG can be any group that is desirably made available in a photographic element in an imagewise fashion. The PUG can be a pnotographic dye or a photographic reagent. A photographic reagent herein is a moiety which upon release further reacts with components in the element, such as a development inhibitor, a development accelerator, a bleach inhibitor, a bleach accelerator, a coupler (e.g. a competing coupler, a color-forming coupler, a DIR coupler), a dye precursor, a dye, a developing agent (e.g. a competing developing agent, a dye-forming developing agent or a silver halide developing agent), a silver complexing agent, a fixing agent, an image toner, a stabilizer, a hardener, a tanning agent, a fogging agent, an ultraviolet radiation absorber, an antifoggant, a nucleator, a chemical or spectral sensitizer or a desensitizer. Such dyes and photographic reagents generally contain a hetero atom having a negative valence of 2 or 3 from Group VA or VIA of the Periodic Table, such as oxygen, sulfur, selenium and nitrogen (e.g., nitrogen in a heterocyclic ring). Such an atom can conveniently serve as the point on the dye or photographic reagent at which the second timing group ($T_2$) is joined.

The PUG can be present in the coupler as a preformed species or it can be present in a blocked form or as a

precursor. For example, a preformed development inhibitor may be attached to the second timing group or the development inhibiting function may be blocked by being the point of attachment to the second timing group. Other examples are (i) a preformed dye attached to the second timing group, (ii) a dye which is blocked so as to shift its spectral absorption attached to the second timing group, or (iii) a leuco dye attached to the second timing group.

Preferred compounds are photographic couplers containing a coupler moiety, a PUG containing a hetero atom from Group VA or VIA of the Periodic Table having a negative valence of 2 or 3, and timing groups ($T_1$ and $T_2$) joining the coupler moiety and the PUG. The first timing group ($T_1$) comprises a nucleophilic group attached to the coupler moiety at a position from which it is capable of being displaced as a result of reaction of the coupler moiety with oxidized color developing agent. The first timing group ($T_1$) also preferably comprises an electrophilic group attached to the second timing group ($T_2$) and capable of being displaced therefrom by the nucleopnilic group after the nucleopnilic group is displaced from the coupler moiety. The coupler also comprises a linking group spatially relating the nucleophilic group and the electrophilic group to enable an intramolecular nucleophilic displacement reaction which cleaves the bond between the second timing group ($T_2$) and the first timing group ($T_1$).

It will be appreciated that in the first timing group, for an intramolecular reaction to occur between the nucleophilic group and the electrophilic group, the groups should be spatially related after cleavage from the coupler, so that they can react with one another. Preferably, the nucleophilic group and the electrophilic group are spatially related within the first timing group so that the intramolecular nucleophilic displacement reaction involves the formation of a 3- to 7-membered ring, most preferably a 5- or 6-membered ring.

It will be further appreciated that for an intramolecular reaction to occur in the aqueous alkaline environment encountered during photographic processing, displacing the second timing group from the first timing group, the thermodynamics should be such and the groups be so selected that the free energy of ring closure plus the bond energy of the bond formed between the nucleophilic group and the electrophilic group is greater than the bond energy between the electrophilic group and the second timing group. Not all possible combinations of nucleophilic group, linking group, electrophilic group and the atoms in the second timing group to which the electrophilic group is attached will yield a thermodynamic relationship favorable to breaking of the bond between the electrophilic group and the second timing group. However, it is within the skill of the an to select appropriate combinations taking the above energy relationships into account.

A typical class of timing group ($T_1$) is represented by the structure:

{Nu-X-E}

wherein:

Nu is a nucleophilic group attached to a position on COUP from which it will be displaced upon reaction of COUP with oxidized color developing agent.
E is an electrophilic group attached to an atom in the second timing group ($T_2$) and is displacable therefrom by Nu after Nu is displaced from COUP; and
X is a linking group for spatially relating Nu and E, upon displacement of Nu from COUP, to undergo an intramolecular nucleophilic displacement reaction with the formation of a 3- to 7-membered ring and thereby release {$T_2$}PUG

Representative Nu groups contain electron rich oxygen, sulfur and nitrogen atoms. Representative E groups contain electron deficient carbonyl, thiocarbonyl, phosphonyl and thiophosphonyl moieties. Other useful Nu and E groups will be apparent to those skilled in the art.

In the following listings of representative Nu and E groups, the groups are oriented so that the lefthand bond of Nu is joined to COUP and the righthand bond of Nu is joined to X, while the lefthand bond of E is joined to X and the righthand bond of E is joined to {$T_2$} PUG

Representative Nu groups include:

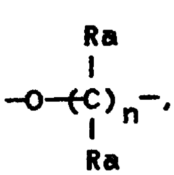 , 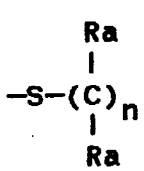 , 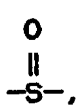 , 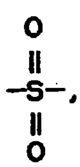 , 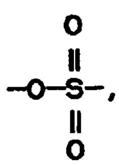 , 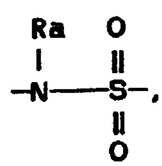 ,

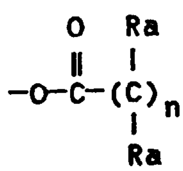, 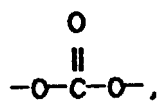, 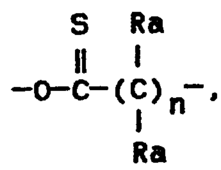, 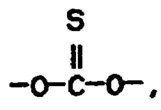,

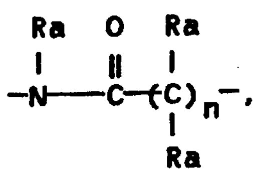, 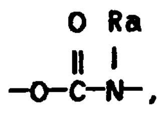, 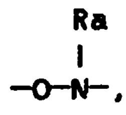, and 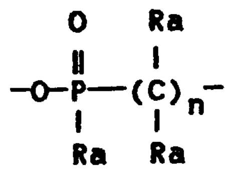

where each Ra is independently hydrogen, alkyl, such as alkyl of 1 to 20 carbon atoms including substituted alkyl such as methyl, ethyl, propyl, hexyl, decyl, pentadecyl, octadecyl, carboxyethyl, hydroxypropyl, sulfonamidobutyl and the like, or aryl, such as aryl of 6 to 20 carbon atoms including substituted aryl such as phenyl, naphthyl, benzyl, tolyl, t-butylphenyl, carboxyphenyl, chlorophenyl, hydroxyphenyl and the like, and n is an integer from 0 to 4 such that the ring formed by Nu, X and E upon nucleophilic attack of Nu upon the electrophilic center in E contains 3 to 7 ring atoms. Preferably Ra is hydrogen, lower alkyl of 1 to 4 carbon atoms or aryl of 6 to 10 carbon atoms.

Representative E groups include:

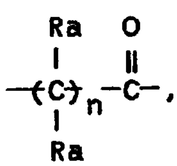, 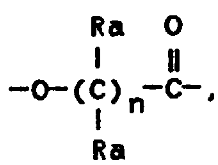, 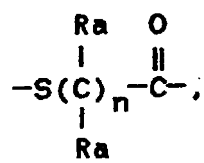, 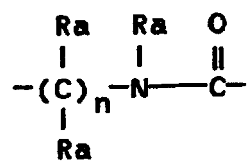,

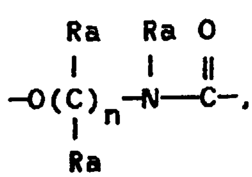, 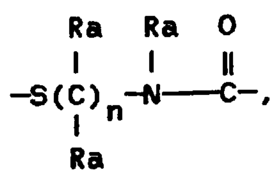, 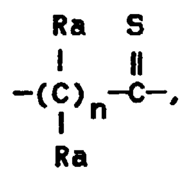, 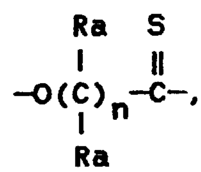,

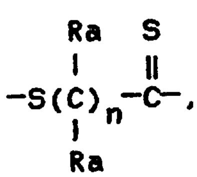, 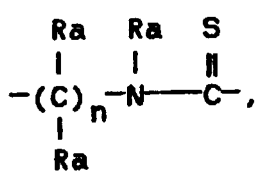, 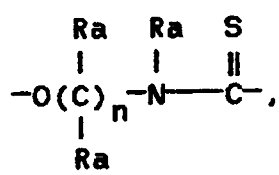, 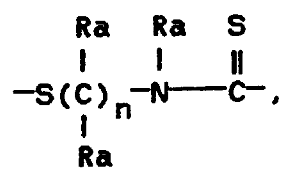,

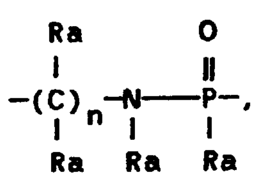, and 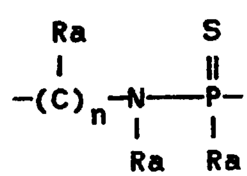

where Ra and n are as defined above.

E is preferably an electrophilic group selected from the group consisting of

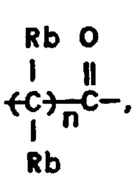, 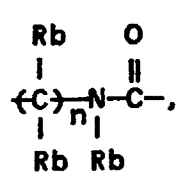, 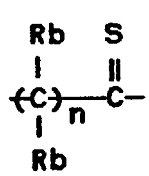 and 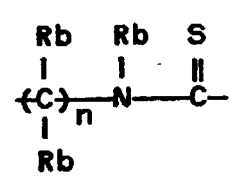

wherein each Rb is independently hydrogen, alkyl, such as alkyl containing 1 to 20 carbon atoms, preferably alkyl containing 1 to 4 carbon atoms, or aryl, such as aryl containing 6 to 20 carbon atoms, preferably aryl containing 6 to 10 carbon atoms; and n is 0 to 4, such that the ring formed upon reaction of the nucleophilic center in Nu with the electrophilic center in E contains 5- or 6-members.

The linking group represented by X can be an acyclic group such as alkylene, such as methylene, ethylene or propylene, or a cyclic group such as an aromatic group, such as phenylene or naphthylene, or a heterocyclic group, such as furan, thiophene, pyridine, quinoline or benzoxazine. Preferably X is alkylene or arylene. The groups Nu and E are attached to X to provide, upon release of Nu from COUP, favorable spatial relationship for nucleophilic attack of the nucleophilic center in Nu on the electrophilic center in E. When X is a cyclic group, Nu and E can be attached to the same or adjacent rings. Aromatic groups in which Nu and E are attached to adjacent ring positions are particularly preferred X groups.

X can be unsubstituted or substituted. The substituents can be those which will modify the rate of reaction, diffusion, or displacement, such as halogen, including fluoro, chloro, bromo, or iodo, nitro, alkyl of 1 to 20 carbon atoms, acyl, such as carboxy, carboxyalkyl, alkoxycarbonyl, alkoxycarbonamido, sulfoalkyl, alkylsulfonamido, and alkylsulfonyl, solubilizing groups, ballast groups and the like, or they can be substituents which are separately useful in the photographic element such as a stabilizer, an antifoggant, a dye (e.g., a filter dye, a solubilized masking dye) and the like. For example, solubilizing groups will increase the rate of diffusion; ballast groups will decrease the rate of diffusion; electron withdrawing groups will decrease the rate of displacement of the second timing group and PUGs which remain attached to X can serve functions such as stabilization, masking and the like.

There follows a listing of patents and publications which describe representative COUP groups useful in the invention. Also listed are structures of preferred COUP, $T_1$, $T_2$, and PUG groups. In these structures Y represents, in the case of a dye forming coupler that is useful with couplers according to the invention, a hydrogen atom or a coupling-off group known in the photographic art. In the case of couplers according to the invention, Y represents $\{T_1\}\{T_2\}$ PUG wherein $T_1$ $T_2$ and PUG are as defined above.

I. COUP's

A. Couplers which form cyan dyes upon reaction with oxidized color developing agents are described in such representative patents and publications as: U.S. Pat. Nos. 2,772,162, 2,895,826, 3,002,836, 3,034,892, 2,474,293, 2,423,730, 2,367,531, 3,041,236 and "Farbkuppler-eine Literatureübersicht," published in Agfa Mitteilungen, Band II, pp. 156-175 (1961).

Preferably such couplers are phenols and naphthols which form cyan dyes on reaction with oxidized color developing agent and have the $\{T_1\}\{T_2\}$ PUG group attached to the coupling position, that is the carbon atom in the 4-position. Structures of preferred such coupler moieties are:

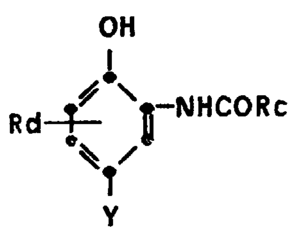

IA–1

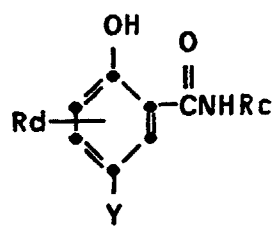

IA–2

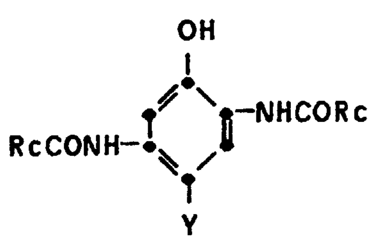

IA–3

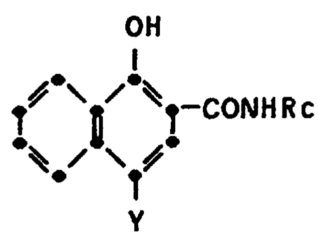

IA–4

where Rc represents a ballast group, and Rd represents one or more halogen (e.g. chloro, fluoro), lower alkyl (e.g. methyl, ethyl, butyl) or lower alkoxy (e.g. methoxy, ethoxy, butoxy) groups.

B. Couplers which form magenta dyes upon reaction with oxidized color developing agent are described in such representative patents and publications as: U.S. Pat. Nos 2,600,788, 2,369,489, 2,343,703, 2,311,082, 3,152,896, 3,519,429, 3,062,653, 2,908,573 and "Fabkupper-eine Literatureũrubersicht," published in Agfa Mitteilungen,Band II, pp. 126-156 (1961).

Preferably, such couplers are pyrazolones and pyrazolotriazoles which form magenta dyes upon reaction with oxidized color developing agents and have the Y, i.e. $\{T_1\}\{T_2\}$ PUG group, attached to the coupling position. Structures of preferred such coupler moieties are:

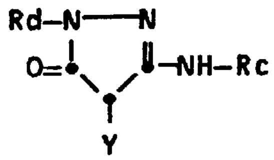

IB-1

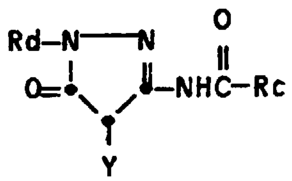

IB-2

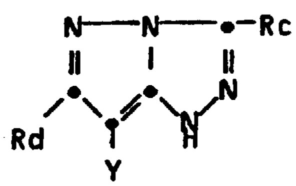

IB-3

where Rc and Rd are chosen independently to be a ballast group, alkyl, substituted alkyl, phenyl or substituted phenyl.

C. Couplers which form yellow dyes upon reaction with oxidized and color developing agent are described in such representative patents and publications as: U.S. Pat. Nos. 2,875,057, 2,407,210, 3,265,506, 2,298,443, 3,048,194, 3,447,928 and "Farbkuppler-eine Literatureübersicht," published in Agfa Mitteilungen, Band II, pp. 112-126 (1961).

Preferably such yellow-dye forming couplers are acylacetamides, such as benzoylacetanilides and pivalylacetanilides, and have the $\{T_1\}\{T_2\}$ PUG group attached to the coupling position, i.e. the active methylene carbon atom.

Structures of preferred such coupler moieties are:

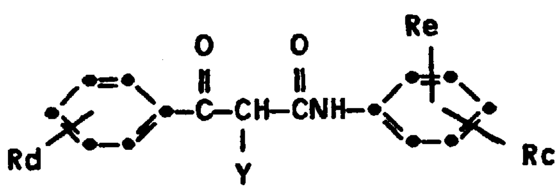

IC-1

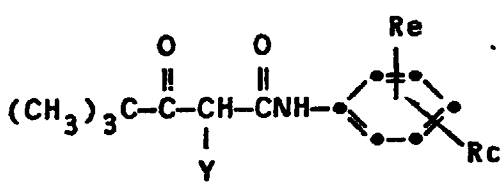

IC-2

where Rc is as defined above and Rd and Re are hydrogen or one or more halogen, lower alkyl, such as methyl and ethyl, or ballast groups, such as alkoxy of 16 to 20 carbon atoms.

D. Couplers which form colorless products upon reaction with oxidized color developing agent are described in such representative patents as: U.K. Patent No. 861,138; U.S. Pat. Nos. 3,632,345, 3,928,041, 3,958,993 and 3,961,959. Preferably such couplers are cyclic carbonyl containing compounds which form colorless products on reaction with oxidized color developing agent and have the $\{T_1\}\{T_2\}$ PUG group attached to the carbon atom in the $\alpha$-position with respect to the carbonyl group.

Structures of preferred such coupler moieties are:

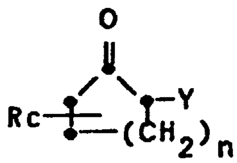

ID-1

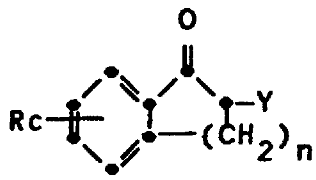

ID-2

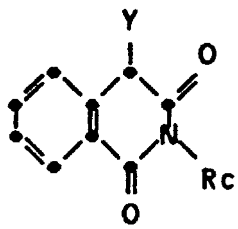

ID-3

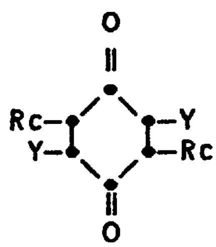

ID-4

where Rc is as defined above and n is 1 or 2.

E. Couplers which form black dyes upon reaction with oxidized color developing agent are described in such representative patents as U.S. Pat. Nos. 1,939,231; 2,181,944; 2,333,106; and 4,126,461; German OLS No. 2,644,194 and German OLS No. 2,650,764.

Preferably such couplers are resorcinols or m-aminophenols which form black or neutral products on reaction with oxidized color developing agent and have the $\{T_1\}\{T_2\}$ PUG group para to a hydroxy group.

Structures of preferred such coupler moieties are:

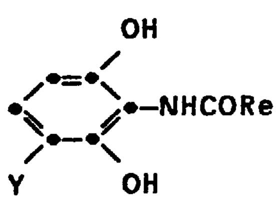

IE-1

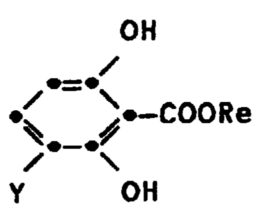

IE-2

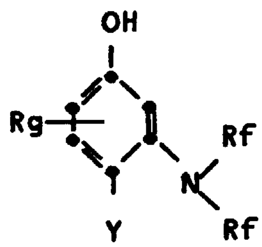

IE–3

where Re is alkyl of 3 to 20 carbon atoms, phenyl or phenyl substituted with hydroxy, halo, amino, alkyl of 1 to 20 carbon atoms or alkoxy of 1 to 20 carbon atoms; each Rf is independently hydrogen, alkyl of 1 to 20 carbon atoms, alkenyl of 1 to 20 carbon atoms, or aryl of 6 to 20 carbon atoms; and Rg is one or more halogen, alkyl of 1 to 20 carbon atoms, alkoxy of 1 to 20 carbon atoms or other monovalent organic groups.

II. First Timing Groups ($T_1$)

Examples of first timing groups ($T_1$) are as follows:

A. Acyclic $T_1$ groups:

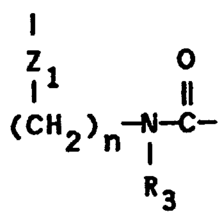

IIA–1

wherein n is 14, preferably 2 or 3, $Z_1$ is

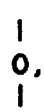, 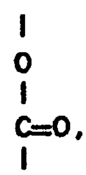, or 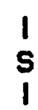

and R3 is hydrogen, alkyl, such as alkyl of 1 to 20 carbon atoms, preferably lower alkyl of 1 to 4 carbon atoms, or aryl, such as aryl of 6 to 20 carbon atoms, preferably aryl of 6 to 10 carbon atoms.

B. Aromatic $T_1$ groups:

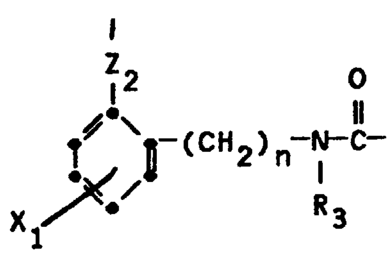

IIB–1

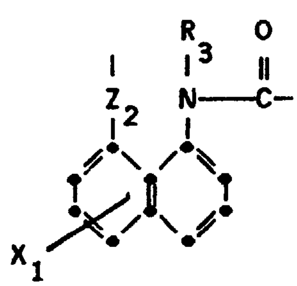

IIB–2

where n is 0 or 1; $Z_2$ is

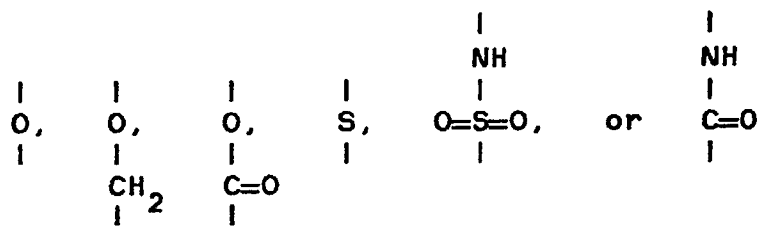

or

$R_3$ is as defined above; and $X_1$ is hydrogen or one or more substituent groups independently selected from cyano, fluoro, chloro, bromo, iodo, nitro, alkyl, such as alkyl of 1 to 20 carbon atoms, a dye, $-OR_4$,$-COOR_4$, $-CONHR_4$, $-NHCOR_4$, $NHSO_2R_4$, $-SO_2NHR_4$ of $SO_2R_4$, where $R_4$ is hdyrogen, alkyl, such as alkyl of 1 to 20 carbon atoms, preferably alkyl of 1 to 4 carbon atoms, or aryl, such as aryl of 6 to 20 carbon atoms, preferably aryl of 6 to 10 carbon atoms.

C. Heterocyclic $T_1$ groups:

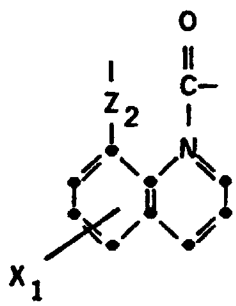

IIC-1

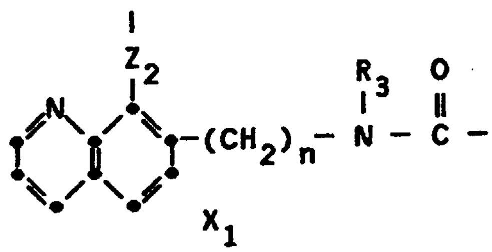

IIC-2

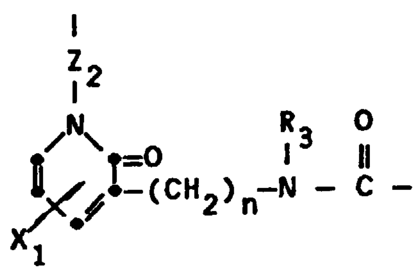

IIC-3

where n is 0 or 1, $Z_2$, $X_1$ and $R_3$ are as defined above.

D. Bis $T_1$ groups:

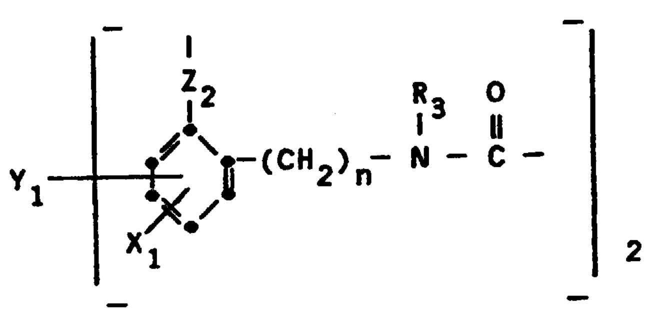

IID-1

where $Y_1$ is a linking group, such as

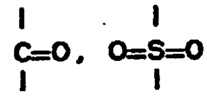

or $-NHSO_2CH_2SO_2NH-$; n is 0 or 1 and $X_1$, $Z_2$ and $R_3$ are as defined above.

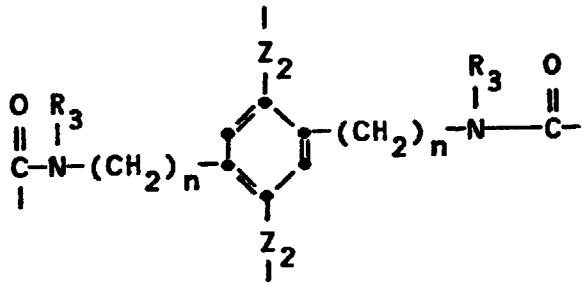

where n is 0 or 1 and $Z_2$, $R_3$ are as defined above.

Such timing groups are described in, for example, U.S. Patent 4,248,962.

III. Second Timing Group ($T_2$)

Examples of the second timing group ($T_2$) are represented by the following formulas:

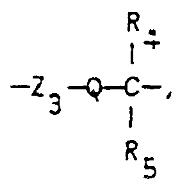

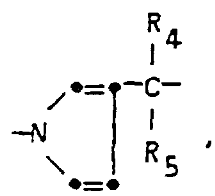

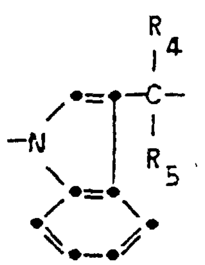

and

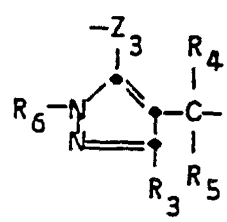

wherein the righthand bond is joined to PUG; the lefthand bond is attached to the first timing group ($T_1$), $Z_3$ is O, S or

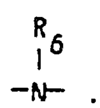

$R_3$, $R_4$, $R_5$ and $R_6$ are individually a hydrogen atom, alkyl or aryl group, and Q is a pyridylene, 1,2- or 1,4-phenylene or naphthylene group. The pyridylene, phenylene or naphthylene can be unsubstituted or substituted by halogen, alkyl, alkoxy, -CN, $-NO_2$, -NHCOR or -COOR wherein R is alkyl.

Such timing groups are described in, for example, U.S. Patent 4,409,323 and Research Disclosure, December 1981, Item No. 21228.

IV. PUG's

A. PUG's which form development inhibitors upon release are described in such representative patents as U.S. Pat. Nos. 3,227,554; 3.384,657; 3,615,506; 3,617,291, 3,733,201 and U.K. Pat. No. 1,450,479. Preferred development inhibitors are iodide and heterocyclic compounds such as mercaptotetrazoles, selenotetrazoles, mercaptobenzothiazoles, selenobenzothiazoles, mercaptobenzoxazoles, selenobenzoxazoles, mercaptobenzimidazoles, selenobenzimidazoles, benzotriazoles and benzodiazoles. Structures of preferred development inhibitor moieties are:

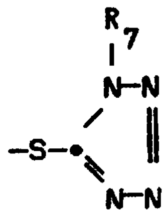

IIIA-1

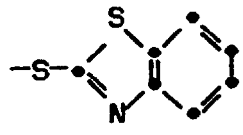

IIIA-2

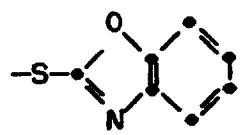

IIIA-3

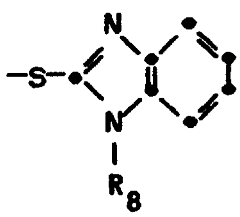

IIIA-4

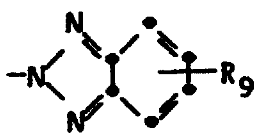

IIIA-5

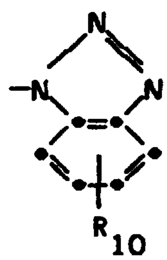

IIIA-6

where $R_7$ and $R_8$ are individually hydrogen, alkyl of 1 to 8 carbon atoms (e.g. methyl, ethyl, butyl), phenyl or substituted phenyl and $R_9$ and $R_{10}$ are individually hydrogen or one or more halogen (e.g. chloro, fluoro, bromo), lower alkyl of 1 to 4 carbon atoms, carboxyl, carboxy esters, such as $-COOCH_3$, $-NH-COOCH_3$, $-SO_2OCH_3$, $-OCH_2CH_2SO_2CH_3$,

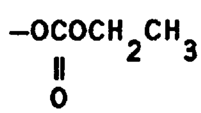 , 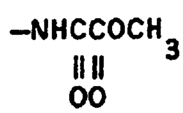

or nitro groups.

B. PUG's which are, or form, dyes upon release:

Suitable dyes and dye precursors include azo, azomethine, azopyrazolone, indoaniline, indophenol, anthraquinone, triarylmethane, alizarin, nitro, quinoline, indigoid and phthalocyanine dyes or precursors of such dyes such as leuco

dyes, tetrazolium salts or shifted dyes. These dyes can be metal complexed or metal complexable. Representative patents describing such dyes are U.S. Pat. Nos. 3,880,658; 3,931,144; 3,932,380; 3,932,381; and 3,942,987. Preferred dyes and dye precursors are azo, azomethine and indoaniline dyes and dye precursors. Structures of some preferred dyes and dye precursors are:

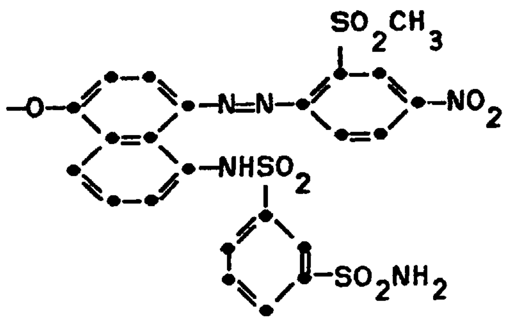

IIIB-1

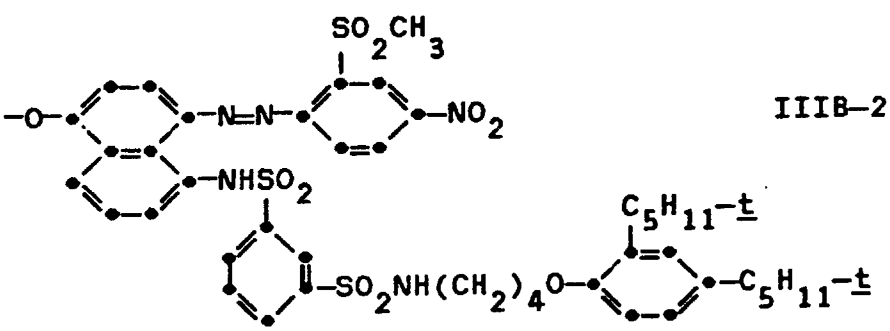

IIIB-2

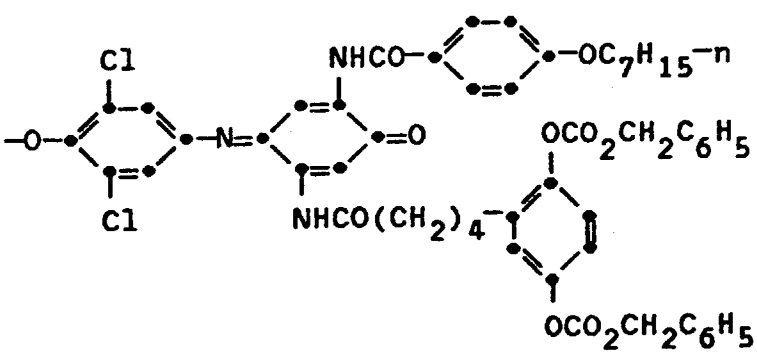

IIIB-3

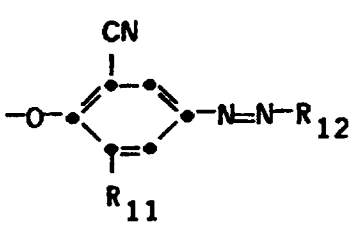

IIIB-4

| $R_{11}$ | $R_{12}$ |
|---|---|
| —H | 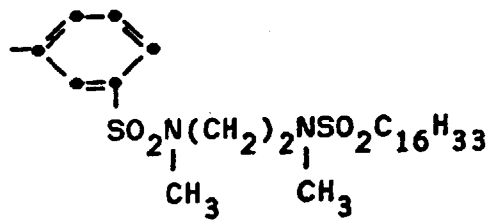 |
| —Cl | 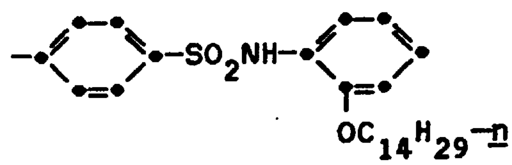 |
| —Cl | 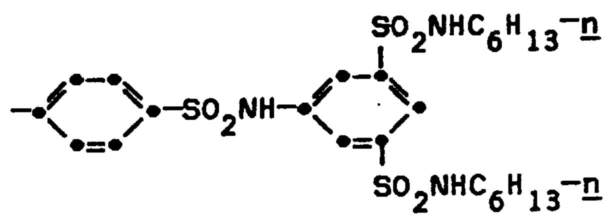 |

C. PUG's which are couplers:

Couplers released can be nondiffusible color-forming couplers, non-color forming couplers or diffusible competing couplers. Representative patents and publications describing competing couplers are: "On the Chemistry of White Couplers," by W. Püschel, Agfa-Gevaert AG Mitteilungen and der Forschungs-Laboratorium der Agfa-Geuaert AG, Springer Verlag, 1954, pp. 352-367; US. Pat. Nos. 2,998,314, 2,808,329, 2,689,793; 2,742,832; German Pat. No. 1,168,769 and British Pat. No. 907,274. Structures of preferred competing couplers are:

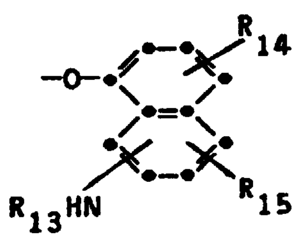

IIIC—1

where $R_{13}$ is hydrogen or alkylcarbonyl, such as acetyl, and $R_{14}$ and $R_{15}$ are individually hydrogen or a solubilizing group, such as sulfo, aminosulfonyl, and carboxy

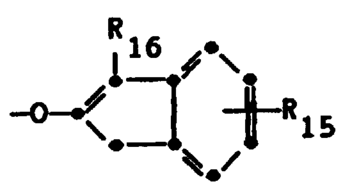

IIIC—2

where $R_{15}$ is as defined above and $R_{16}$ is halogen, aryloxy, arylthio, or a development inhibitor, such as a mercaptotetrazole, such as phenylmercaptetrazole or ethyl mercaptotetrazole.

D. PUG's which form developing agents:

Developing agents released can be color developing agents, black-and-white developing agents or cross-oxidizing developing agents. They include aminophenols, phenylene diamines, hydroquinones and pyrazolidones. Representative patents are: U.S. Pat. Nos. 2,193,015; 2,108,243; 2,592,364; 3,656,950; 3,658,525; 2,751,297; 2,289,367; 2,772,282; 2,743,279; 2,753,256; and 2,304,953.

Structures of preferred developing agents are:

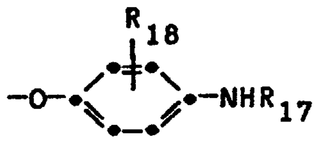

IIID-1

where $R_{17}$ is hdyrogen or alkyl of 1 to 4 carbon atoms and $R_{18}$ is hydrogen or one or more halogen (e.g. chloro, bromo) or alkyl of 1 to 4 carbon atoms (e.g. methyl, ethyl, butyl) groups.

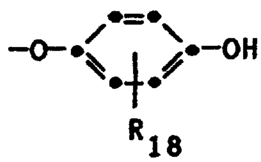

IIID-2

where $R_{18}$ is as defined above.

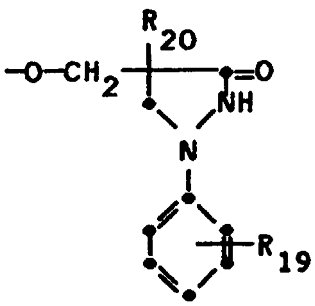

IIID-3

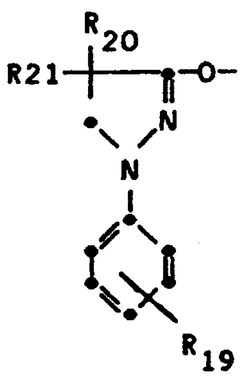

IIID-4

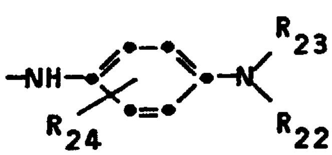

IIID-5

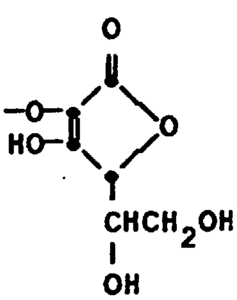

IIID-6

where $R_{19}$ is hydrogen or alkyl of 1 to 4 carbon atoms and $R_{20}$, $R_{21}$, $R_{22}$, $R_{23}$ and $R_{24}$ are individually hydrogen, alkyl

of 1 to 4 carbon atoms (e.g. methyl, ethyl) lower hydroxyalkyl of 1 to 4 carbon atoms (e.g. hydroxymethyl, hyroxyethyl) or lower sulfoalkyl.

E. PUG's which are bleach inhibitors:

Representative patents are U.S. Pat. Nos. 3,705,801; 3,715,208; and German OLS No. 2,405,279. Structures of preferred bleach inhibitors are:

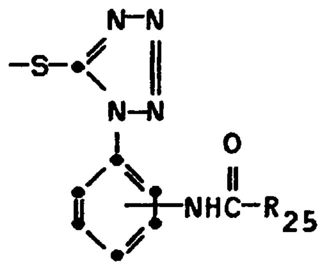

IIIE-1

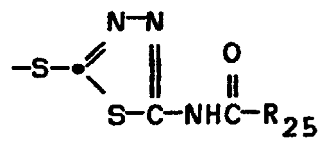

IIIE-2

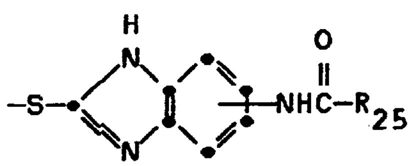

IIIE-3

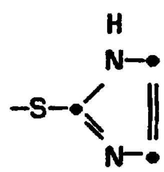

IIIE-4

where $R_{25}$ is an alkyl group of 6 to 20 carbon atoms.

F. PUG's which are bleach accelerators:

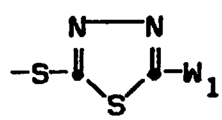

IIIF-1

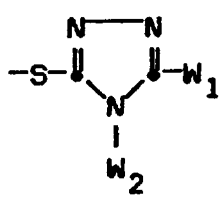

IIIF-2

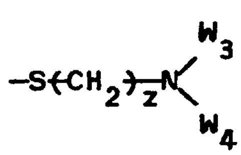

IIIF-3

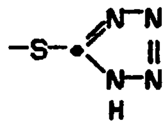

IIIF-4

$-SCH_2CH_2COOH$ IIIF-5

wherein $W_1$ is hdyrogen, alkyl, such as ethyl and butyl, alkoxy, such as ethoxy and butoxy, or alkylthio, such as ethylthio and butylthio, for example containing 1 to 6 carbon atoms, and which may be unsubstituted or substituted; $W_2$ is hydrogen, alkyl or aryl, such as phenyl; $W_3$ and $W_4$ are individually alkyl, such as alkyl containing 1 to 6 carbon atoms, for example ethyl and butyl; z is 1 to 6.

The timing groups ($T_1$ and $T_2$) and PUG are selected and prepared to adjust to the activity of the adjoining carder moiety, particularly a coupler moiety and the other groups of the coupler in order to optimize release of the PUG for its intended purpose. Accordingly, PUG groups of differing structural types are useful which enable timing groups having a range of activities. Various properties, such as pKa, are also usefully considered in optimizing the selection of optimum groups for a particular purpose. An example of such a selection could involve, for instance, a benzotriazole moiety as a PUG. Such a benzotriazole moiety can be released too slowly for some intended purposes from a timing group which involves a quinone-methide release mechanism and yet too quickly from a timing group which involves an intramolecular nucleophilic displacement mechanism; however, the benzotriazole moiety can be modified from

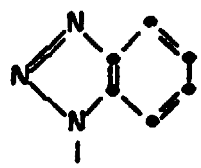

to

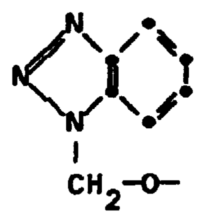

in order to modify the rate at which the benzotriazole moiety is cleaved from the timing group ($T_2$). Another illustration of modifying the PUG involves changing, for example, a mercaptotetrazole moiety from

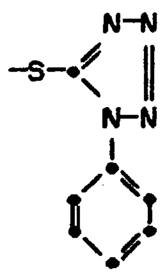

to

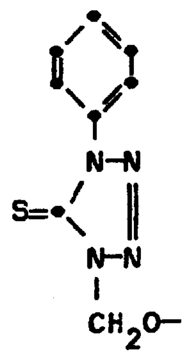

at elevated pH, such as above about pH 10, wherein the $-CH_2O-$ portion of the group hydrolyzes rapidly leaving the remainder of the PUG free for its intended purpose.

A preferred compound A is a coupler represented by the formula:

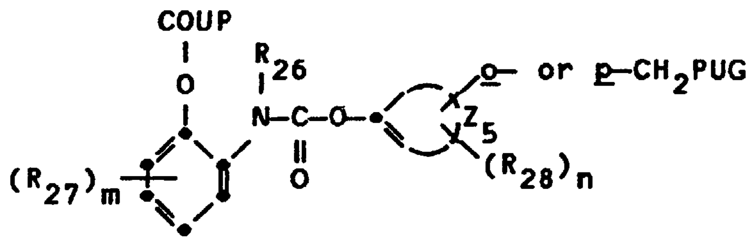

wherein:

$R_{26}$ is substituted or unsubstituted alkyl, such as substituted or unsubstituted alkyl containing 1 to 20 carbon atoms, for example methyl, ethyl, propyl, butyl or eicosyl; substituted or unsubstituted aryl, such as substituted or unsubstituted aryl containing 6 to 20 carbon atoms, for example, phenyl, hydroxyphenyl, aryloxyphenyl, and alkoxyphenyl;

$R_{27}$ is hydrogen or a substituent which does not adversely affect timing of release of the other portions of the compound, such as alkyl, for example alkyl containing 1 to 12 carbon atoms, alkylsulfonyl, for example $-SO_2C_{12}H_{25}$, nitro, alkoxy, halogen, sulfonamido, sulfamoyl or cyano;

$R_{28}$ is hydrogen or a substituent that advantageously influences the timing of release of PUG, such as alkyl containing 1 to 5 carbon atoms, for example methyl or butyl, nitro, halogen, cyano or alkoxy containing 1 to 5 carbon atoms;

COUP is a coupler moiety substituted in the coupling position by the remainder of the coupler;

PUG is a photographically useful group;

m and n are individually 0, 1 or 2;

$Z_5$ represents the atoms necessary to complete a pyridine, pyrazole, benzene, or naphthalene nucleus.

The photographic couplers can be incorporated in photographic elements and/or in photographic processing solutions, such as developer solutions, so that upon development of an exposed photographic element they will be in reactive association with oxidized color developing agent. Coupler compounds incorporated in photographic processing solutions should be of such molecular size and configuration that they will diffuse through photographic layers with the processing solution. When incorporated in a photographic element, as a general rule, the coupler compounds should be nondiffusible, that is they should be of such molecular size and configuration that they will not significantly diffuse or wander from the layer in which they are coated.

Photographic elements of this invention can be processed by conventional techniques in which color forming couplers and color developing agents are incorporated in separate processing solutions or compositions or in the element.

Photographic elements in which compound A is incorporated can be a simple element comprising a support and a single silver halide emulsion layer or they can be multilayer, multicolor elements. The compound A can be incorporated in at least one of the silver halide emulsion layers and/or in at least one other layer, such as an adjacent layer, where they will come into reactive association with oxidized color developing agent which has developed silver halide in the emulsion layer. The silver halide emulsion layer can contain or have associated with it, other photographic coupler compounds, such as dye-forming couplers, colored masking couplers, and/or competing couplers. These other photographic couplers can form dyes of the same or different color and hue as the photographic couplers of this invention. Additionally, the silver halide emulsion layers and other layers of the photographic element can contain addenda conventionally contained in such layers.

A typical multilayer, multicolor photographic element can comprise a support having thereon a red-sensitive silver halide emulsion unit having associated there-with a cyan dye image providing material, a green-sensitive silver halide emulsion unit having associated therewith a magenta dye image providing material and a blue-sensitive silver halide emulsion unit having associated therewith a yellow dye image-providing material, at least one of the silver halide emulsion units having associated therewith a photographic coupler of the invention. Each silver halide emulsion unit can be composed of one or more layers and the various units and layers can be arranged in different locations with respect to one another.

The couplers can be incorporated in or associated with one or more layers or units of the photograpic element. If $\{T_1\}\{T_2\}$ PUG and/or $\{T_2\}$ PUG and/or PUG are diffusible moieties, the layer or unit affected by PUG can be controlled by incorporating in appropriate locations in the element a scavenger layer which will confine the action of $\{T_2\}$ PUG and/or PUG to the desired layer or unit. At least one of the layers of the photographic element can be, for example, a mordant layer or a barrier layer.

The light sensitive silver halide emulsions can include coarse, regular or fine grain silver halide crystals or mixtures thereof and can be comprised of such silver halides as silver chloride, silver bromide, silver bromoiodide, silver chlorobromide, silver chloroiodide, silver chlorobromoiodide and mixtures thereof. The emulsion can be negative-working or direct-positive emulsions They can form latent images predominantly on the surface of the silver halide grains or predominantly on the interior of the silver halide grains. They can be chemically and spectrally sensitized. The emulsions typically will be gelatin emulsions although other hydrophilic colloids are useful. Tabular grain light sensitive silver halides are particularly useful such as described in Research Disclosure, January 1983, Item No. 22534 and U.S. Patent 4,434,226.

The support can be any support used with photographic elements. Typical supports include cellulose nitrate film, cellulose acetate film, polyvinylacetal film, polyethylene terephthalate film, polycarbonate film and related films or resinous materials as well as glass, paper, metal and the like. Typically, a flexible support is employed, such as a polymeric film or paper support. Paper supports can be acetylated or coated with baryta and/or an α-olefin polymer, particularly a polymer of an α-olefin containing 2 to 10 carbon atoms such as polyethylene, polypropylene, ethylene-butene copolymers and the like.

The photographic couplers can be used in photographic elements in the same way as photographic couplers which release PUGs have previously been used in photographic elements. However, because of the improved ability to control the release of the PUG, the couplers permit enhanced effects or more selective effects than heretofore were possible. In addition, the couplers can be employed in applications where conventional couplers have previously been employed and a separate component was employed to provide a PUG.

Depending upon the nature of the particular PUG, the couplers can be incorporated in a photographic element for different purposes and in different locations.

When the PUG released from the coupler is a development inhibitor, the coupler can be employed in a photographic element like couplers which release development inhibitors have been used in the photographic art. The couplers which release a development inhibitor can be contained in, or in reactive association with, one or more of the silver halide emulsion units in a color photographic element. If the silver halide emulsion unit is composed of more than one layer, one or more of such layers can contain the coupler. The layers can contain other photographic couplers conventionally used in the art. The coupling reaction using couplers as described can form dyes of the same color as the color forming coupler(s) in the layer or unit, it can form a dye of a different color, or it can result in a colorless or neutral reaction product. The range of operation between layers of the development inhibitor released from the coupler can be controlled by the use of scavenger layers, such as a layer of fine grain silver halide emulsion. Scavenger layers can be in various locations in an element containing couplers as described. They can be located between layers, between the layers and the support, or over all of the layers.

Couplers as described which release development inhibitors can enhance the effects heretofore obtained with DIR couplers since they can release a development inhibitor at a distance from the point at which oxidized color developing agent reacted with the coupler, in which case they can provide, for example, enhanced interlayer interimage effects. Thus, the couplers can be employed to provide a degree of control over the effects obtainable from DIR couplers which heretofore could not be attained.

Photographic couplers as described which release bleach inhibitors or bleach accelerators can be employed in the ways described in the photographic art to inhibit the bleaching of silver or accelerated bleaching in areas of a photographic element.

Photographic couplers as described which release a dye or dye precursor can be used in processes where the dye is allowed to diffuse to an integral or separate receiving layer to form a desired image. Alternatively, the dye can be retained in the location where it is released to augment the density of the dye formed from the coupler from which it is released or to modify or correct the hue of that dye or another dye. In another embodiment, the dye can be completely removed from the element and the dye which was not released from the coupler can be retained in the element as a

color correcting mask.

Couplers as described can be employed to release another coupler and the PUG. If the released coupler is a dye-forming coupler it can react with oxidized developing agent in the same or an adjacent layer to form a dye of the same or a different color or hue as that obtained from the primary coupler. If the released coupler is a competing coupler it can react with oxidized color developing agent in the same or an adjacent layer to reduce dye density.

Photographic couplers as described in which the PUG is a developing agent can be used to release a developing agent which will compete with the color forming developing agent, adn thus reduce dye density. Altematively, the couplers can provide, in an imagewise manner, a developing agent which because of such considerations as activity would not desirably be introduced into the element in a uniform fashion.

In chemical systems requiring timed release of a moiety as described herein, the release mechanisms can be initiated by any means that initiates cleavage of the first timing group from the carrier moiety. Depending on the particular carrier compound, the particular timing groups, the desired end use of the active moiety, the release mechanism can, for example, be initiated by reaction of the carrier compound with radiation, enzymes, moisture, acid or base, and/or oxidized reducing agent.

Compound A as described can be prepared by methods known in the organic compound synthesis art. Typically, the couplers are prepared by first attaching the $T_1$ and $T_2$ groups to the appropriate coupler moiety, or a derivative of the coupler moiety. The product is then reacted with an appropriate derivative of the PUG to form the desired coupler. Known reactions are employed to perform these steps. The following examples illustrate the way in which these steps can be performed using specific reactants and reactions.

The following compounds illustrate methods of preparing the compounds.

In these examples Cp is the coupler moiety:

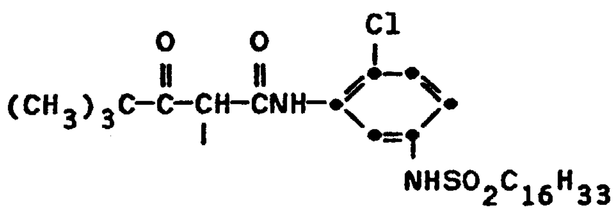

and the designation PMT means the moiety:

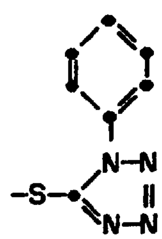

Synthesis Example A:

a. Preparation of Intermediate Compound A-1

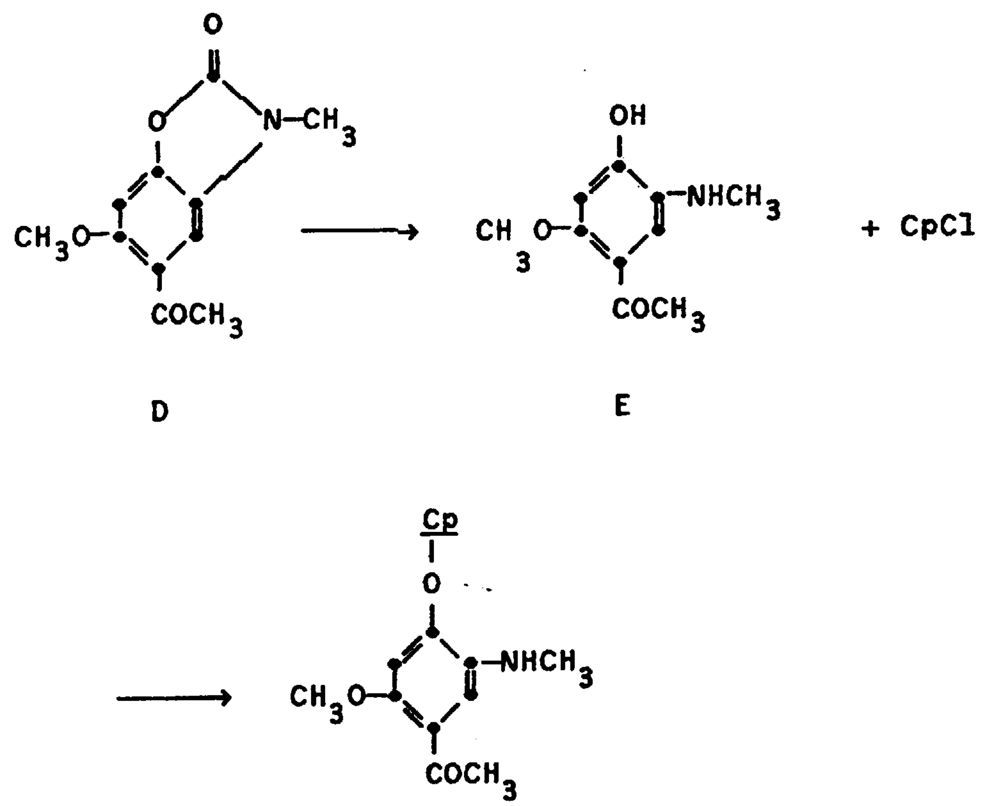

D E

A-1

A slurry of 69 g (0.31 mol) of Compound D in a solution of 51.5 g (0.78 mol) potassium hydroxide in 300 ml water was heated to boiling for 15 minutes. Most of the solid dissolved. Addition of 7.5 g more potassium hydroxide in 50 ml water dissolved most of the remaining solid. The solution was filtered to remove some insoluble impurities.

Careful hydrochloric acid treatment of the ice-cooled filtrate resulted in the evolution of carbon dioxide, and adjusting the pH to 6-7 with ammonium hydroxide gave a precipitate. After washing with water, then a 4:1 diethyl ether/ligroin mixture, 60.3 g of Compound E was obtained as a yellow-green solid.

A solution of 6.3 g (54 mmol) 1,1,3,3,-tetramethylguanidine was added dropwise with stirring over 10 minutes to a solution of 16.2 g (27 mmol) α-chloro-α-pivalyl-2-chloro-5-(n-hexadecylsulfonamide)acetonilide (Cp-C1) and 5.3 g (27 mmol) Compound E in 40 ml acetonitrile. After 1 hour the mixture was partitioned between ethyl acetate and 10% aqueous hydrochloric acid. The organic layer was washed with brine, dried, concentrated , and purified via silica gel chromatography to yield 18.3 g A-1 as a reddish-yellow oil with the expected nmr spectrum.

b. Preparation of Intermediate Compound B-1:

To 50 ml acetyl chloride ice-cooled to 10° was added portionwise with stirring 5.1 g (40 mmol) p-hydroxybenzyl alcohol. After stirring overnight the mixture was concentrated, dissolved in methylene chloride, washed with 5% sodium bicarbonate, water, and dried over magnesium sulfate. The resulting p-acetoxybenzyl chloride in 50 ml methylene chloride was combined at room temperature with a solution of 8.2 g (41 mmol) 1-phenyl-1H-tetrazole-5-thiol sodium salt (NaPMT) in 50 ml water. Work-up and recrystallization in turn from toluene and from isopropanol yielded 7.0 g colorless crystals, m.p. 126-8°, p-$HOC_6H_4CH_2$PMT. To an ice cold solution containing 5.0 g (21 mmol) of this phenolic product in 25 ml tetrahydrofuran and 2.5 g (21 mmol) N,N-dimethylaniline was added with stirring a 12% solution in toluene containing 4.1 g (41 mmol) phosgene. Work-up gave 7.25 g of Compound B-1 represented by the structure:

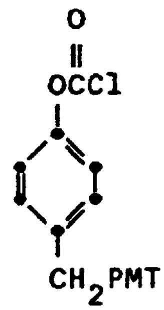

as a blue oil containing a trace of toluene.

c. Preparation of Compound 1 represented by the structure:

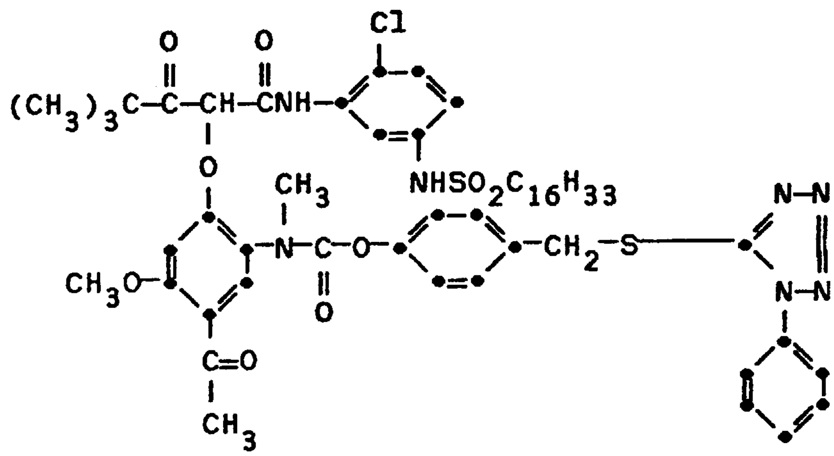

**Compound 1**

To a stirred solution of 6.0 g (8 mmol) A-1 in 20 ml tetrahydrofuran was added under nitrogen 0.97 g (8 mmol) N, N-dimethylaniline and 2.8 g (8 mmol) chloroformate ester B-1. After stirring one hour at room temperature, the reaction mixture was diluted with diethyl ether, washed in turn with saturated sodium chloride solution (brine), 10% aqueous hydrochloric acid, brine, then dried over magnesium sulfate and concentrated to 9.0 g of dark oil. Purification by silica gel chromatography gave 3.1 g light yellow glassy solid melting at 59 to 60°C, with the nmr, infrared, and mass spectra expected for compound 1.

Synthesis Example B-Preparation of Compound 2 represented by the structure:

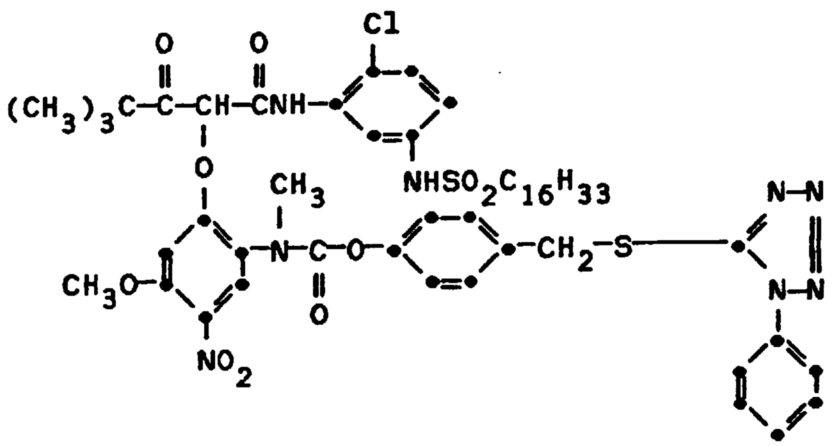

**Compound 2**

To a stirred solution of 8.3 g (11.0 mmol) A-2 represented by the structure:

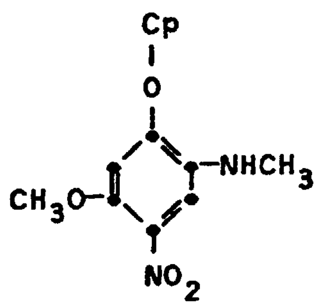

and 1.4 ml (11.0 mmol) N,N-dimethylaniline in 20 ml tetrahydrofuran was added under nitrogen a solution of 3.6 g (10.5 mmol) B-1. After one hour stirring the mixture was partitioned between diethyl ether and 10% aqueous hydrochloric acid. The organic layer was washed with 5% sodium carbonate solution, 5% aqueous hydrochloric add, brine, then dried and concentrated to give 9.3 g of yellow oil. Purification by silica gel chromatography and washing with 5% aqueous sodium hydroxide yielded 3.4 g light yellow solid, m.p. 81-2°, with the elemental analysis, infrared, nmr, and mass spectra expected for compound 2.

Synthesis Example C-Preparation of compound 3 represented by the structure:

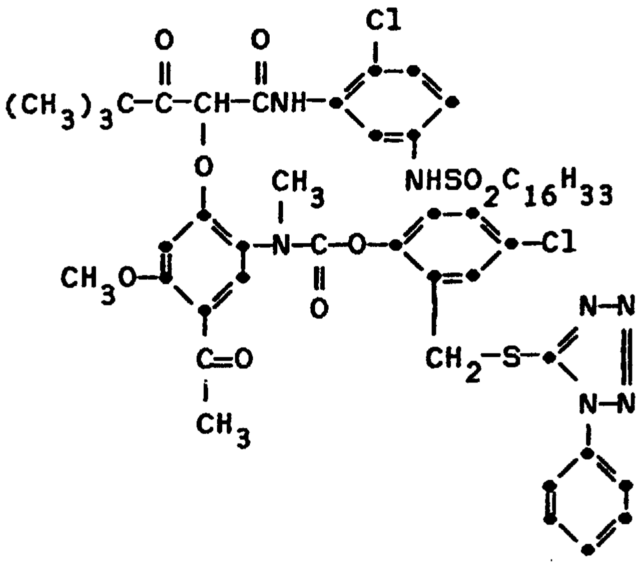

Compound 3

Using the procedure of Synthesis Example A, 9.0 g (12 mmol) of compound A-1 was combined with 5.4 g (14 mmol) of B-2 represented by the structure:

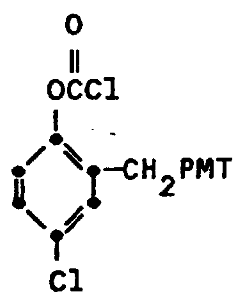

to give, after purification, 3.6 g white solid compound 3, m.p. 79-80°, which was confirmed by elemental analysis and infrared spectra.

Synthesis Examples D and E-

Similar combinations of A-3 represented by the structure:

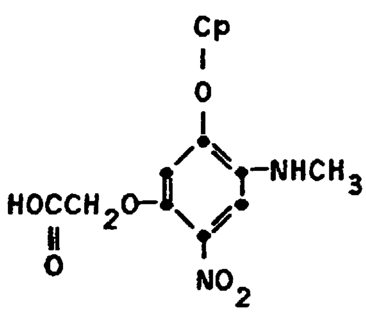

with B-2 and A-3 with B-1, according to the methods of Synthesis Examples A, B and C gave compounds 4 and 5, respectively, represented by the following structures:

Compound 4:

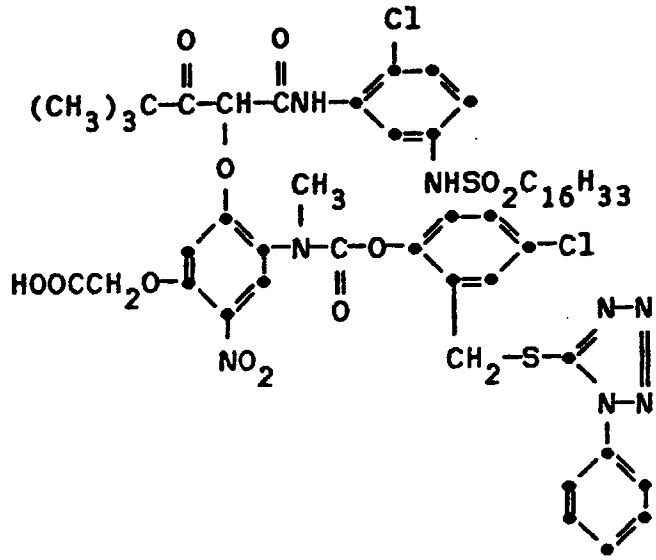

Compound 5:

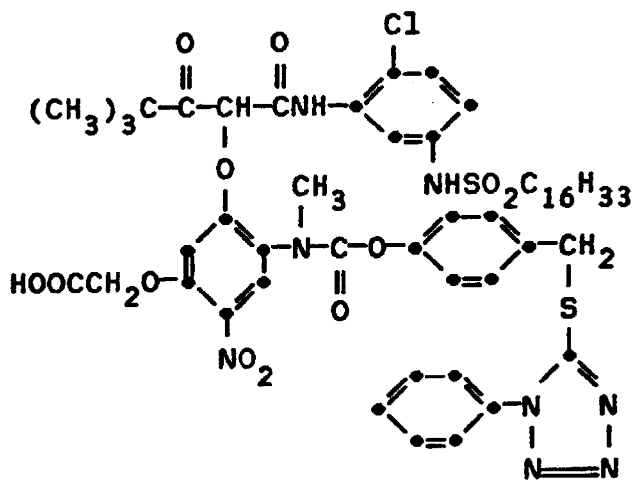

The following compounds 6 and 7 can also be prepared by methods like those described in Synthesis Examples A, B and C:

Compound 6:

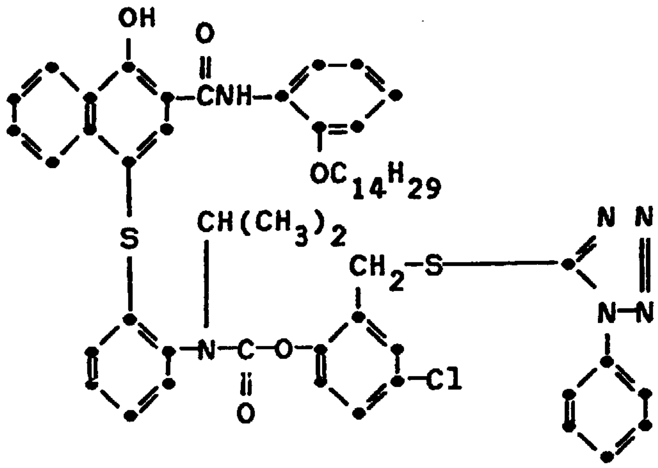

Compound 7:

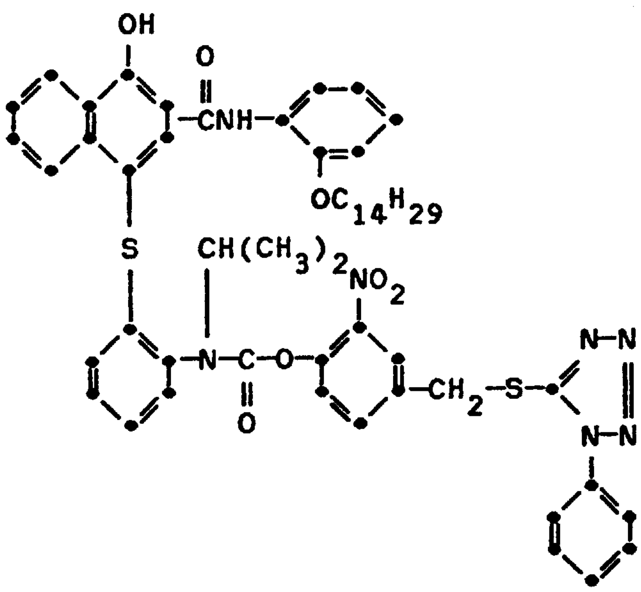

Other representative methods of synthesis are illustrated by the following structural reactions:

I.

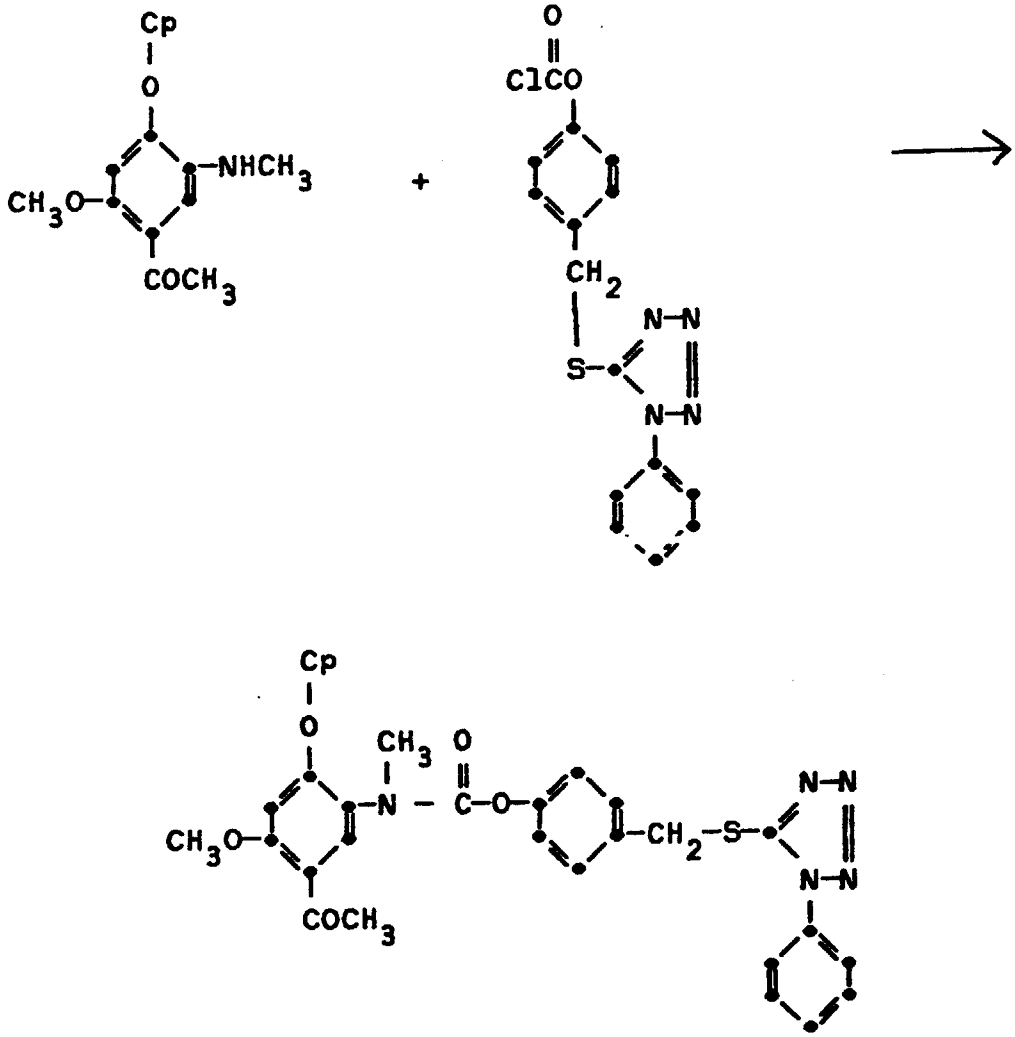

wherein Cp represents a coupler moiety.

II.

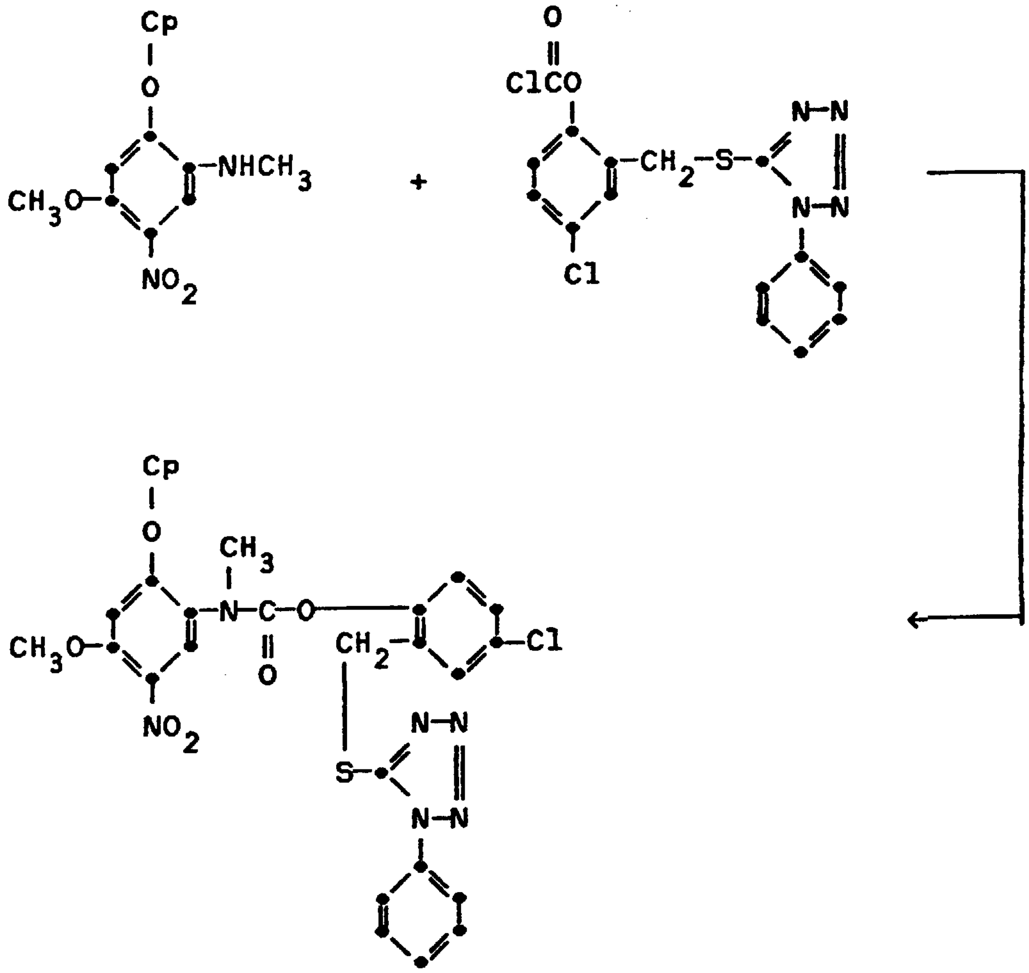

The following examples further illustrate the invention.

Example 1 -

This illustrates the invention.

To demonstrate sharpness and interimage effects a photographic element format was employed in which a green-sensitive AgBrI "causer" gelatino emulsion layer provided a yellow image and an underlying red-sensitive AgBrI "receiver" gelatine emulsion layer provided a magenta image. The term causer gelatine emulsion herein means the layer from which a development inhibitor moiety is released. The term receiver gelatine emulsion layer herein means the layer upon which the development inhibitor acts. Interlayer interimage effects are described in, for example, Barr et al, Photographic Science andEngineering, Volume 13, No. 2, March-April, 1969, pages 78-80. Color photographic materials were prepared according to the following schematic layer structure (numerical values denote coating coverages in $mg/m^2$):

| | |
|---|---|
| Overcoat: | Gelatin-2500; Gelatin hardener 1.75% to total gelatin |
| Causer Layer: | Green-sensitive AgBrI-1600; Gelatin-2400; Yellow dye-forming coupler and image modifying coupler (see Table 3) |
| Interlayer: | Antistain agent 2,5-Didodecylhydroquinone-115; Gelatin-620 |
| Receiver Layer: | Red-sensitive AgBrI-1600, Gelatin-2400; Magenta dye-forming coupler-650 |
| Film Support: | With antihalation gray silver-324; Gelatin-2452; Antistain agent-15 |

The hardener was bis(vinylsulfonylmethyl)-ether and the silver bromoiodide (coating weight is that of silver) was a 6.4% iodide emulsion of 0.5µ average grain size chemically sensitized with sulfur and gold. The yellow dye-forming

coupler was dispersed in half its weight of dibutyl phthalate, the magenta coupler in half its weight of tricresyl phosphate, and each image modifying coupler in twice its weight of diethyl lauramide. The yellow dyeforming coupler was either Coupler Y-1 or Y-2 as designated in Table 3:

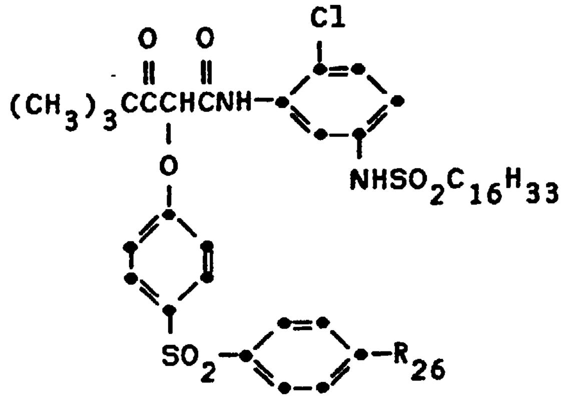

Y-1 $R_{26}$ = $-OCH_2C_6H_5$
Y-2 $R_{26}$ = -OH

The magenta dye-forming coupler was:

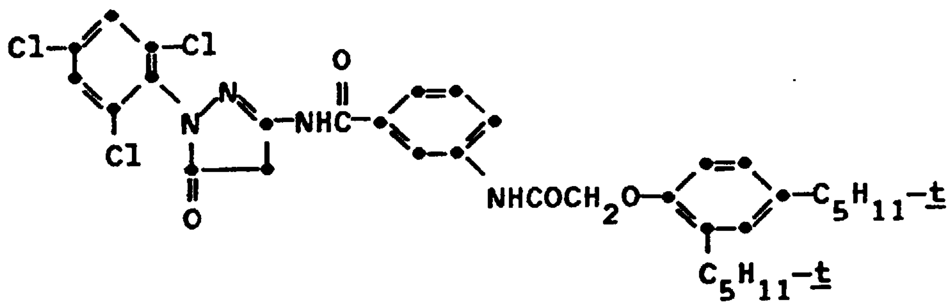

For interimage evaluation requiring image development in both the causer and receiving layers, the samples were exposed through a graduated-densty test object and Wratten 12 (minus blue) filter. Wratten is a trademark of Eastman Kodak Co., U.S.A. For sharpness, evaluated by calculating CMT acutance values for 16 mm movie film (CMT-16) or 35 mm slide film (CMT-35)*, exposures were made through a Wratten 99 (green) filter. The materials were then processed at 38°C as follows:

| | Time in Minutes |
|---|---|
| Color Developer | 2-3/4 |
| Stop (5% Acetic Acid) | 2 |
| Wash | 2 |
| Bleach (fe(CN)$_6$) | 2 |
| Wash | 2 |
| Fix | 2 |
| Wash | 2 |

*This technique and the Cascaded Modulation Transfer (CMT) acutance are discussed in an article entitled: "An Improved Objective Method for Rating Picture Sharpness: CMT Acutance, " by R. G. Gendron, Journal of the SMPTE, 82, pages 1009-12 (December, 1973).

| Color developer composition | g/l |
|---|---|
| $K_2SO_3$ | 2.0 |
| 4-Amino-3-methyl-N-ethyl-N-β-hydroxyethylaniline sulfate | 3.35 |

(continued)

| Color developer composition | g/l |
|---|---|
| $K_2CO_3$ | 30.0 |
| KBr | 1.25 |
| KI | 0.0006 |
| Adjusted to pH 10 | |

The oxidized color developing agent generated by development of exposed silver halide couples with adjacent couplers to produce dye and release $\{T_1\}\{T_2\}$ PMT from each image modifying coupler in the causer layer.

$\{T_1\}\{T_2\}$ PMT subsequently releases PMT. The effects of this inhibitor released from the modifier coupler can be measured by percent gamma repression in the causer (C) or receiver (R) layers.

$$C = 100 \frac{\gamma_0-\gamma}{\gamma_0}$$

image of causer layer

$$R = 100 \frac{\gamma_0-\gamma}{\gamma_0}$$

image of receiver layer

$\gamma_0$ = layer contrast without modifier
$\gamma$ = layer contrast with modifier

The C-R value indicates the relative inhibition effects occurring in the causer and receiver layers.

Similarly, the effect of inhibitor release when only the causer layer is exposed can be measured by percent density repression (% ΔD) in that layer.

$$\% \Delta D = 100 \frac{D_0-D}{D_0}$$

where D and $D_0$ are the net densities (image density minus fog density) for samples with and without incorporated modifier coupler, respectively.

Interimage Effects

In Table 3A comparison couplers are shown to give a wide range of interimage effects when both causer and receiver layers are exposed. All three (C-1, C-2, C-3) have a single timing group ($T_1$) releasing the inhibitor via formation of a 6-membered ring. Variation in the C-R value from +28 to -16 was controlled by attaching to the nitrogen atom either large hydrophobic or hydrophilic substituents, respectively. A small R substituent resulted in a C-R value closer to zero.

Comparison coupler C-1 herein is represented by the structure:

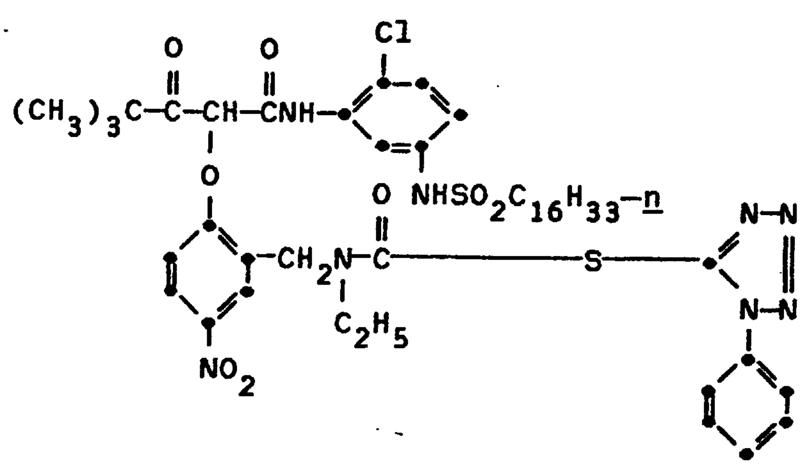

Comparison coupler C-2 herein is represented by the structure:

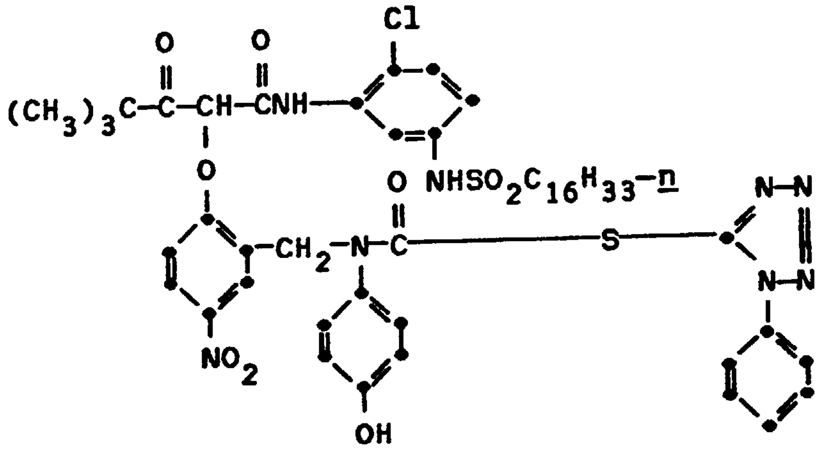

Comparison coupler C-3 herein is represented by the structure:

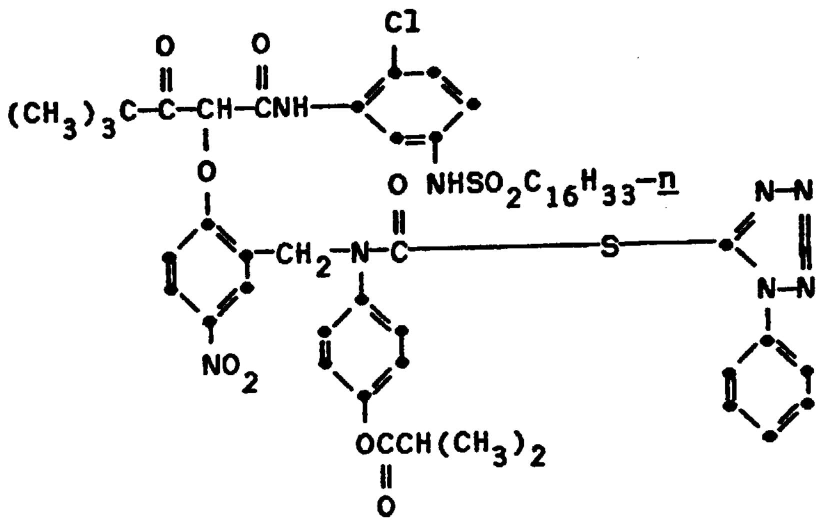

Table 3

| Example No. | Sample | Modifier (Coupler Level) | Interimage Effects | | | | | CMT-16 Sharpness** | | |
|---|---|---|---|---|---|---|---|---|---|---|
| | | | YC | YR | C | R | C-R | c | r | c-r |
| | A. Y-1 Image Coupler | | | | | | | | | |
| - | 1. | None (control) | 1.94 | 1.65 | - | - | - | 91.2 | 87.4 | 3.8 |
| 1 | 2. | C-1 (4.8) | 0.91 | 0.80 | 53 | 52 | 1 | 86.2 | 91.6 | 4.6 |
| 2 | 3. | C-2 (4.8) | 1.08 | 0.66 | 44 | 60 | -16 | 95.5 | 93.7 | 1.8 |
| 3 | 4. | C-2 (9.7) | 0.70 | 0.40 | 64 | 76 | -12 | 99.2 | 97.4 | 1.8 |
| 4 | 5. | C-3 (9.7) | 0.58 | 0.95 | 70 | 42 | 28 | 94.1 | 88.5 | 5.6 |
| 5 | 6. | 2 (9.7) | 0.87 | 0.85 | 55 | 49 | 6 | 96.3 | 91.0 | 5.3 |
| 6 | 7. | 2 (14.6) | 0.66 | 0.72 | 66 | 56 | 10 | 97.7 | 92.0 | 5.7 |
| 7 | 8. | 3 (14.5) | 1.32 | 1.13 | 32 | 32 | 0 | 93.1 | 87.7 | 5.4 |

**Sharpness as CMT for the causer (c) or receiver (r) layer on 1/60 sec. neutral exposure.

Table 3 (continued)

| Example No. | Sample | Modifier (Coupler Level) | Interimage Effects | | | | | CMT-16 Sharpness** | | |
|---|---|---|---|---|---|---|---|---|---|---|
| | B. Y-1 Image Coupler | | | | | | | | | |
| - | 9. | None (control) | 1.65 | 1.31 | - | - | - | 89.6 | 87.0 | 2.6 |
| 8 | 10. | C-1 (2.4) | 1.17 | 0.89 | 29 | 32 | -3 | 92.4 | 89.1 | 3.3 |
| 9 | 11. | 1 (8.5) | 1.04 | 0.84 | 37 | 36 | 1 | 91.7 | 89.0 | 3.7 |
| | C. Y-1 Image Coupler | | | | | | | | | |
| - | 12. | None (Control) | 1.86 | 1.60 | - | - | - | 89.5 | 85.6 | 3.9 |
| 10 | 13. | C-1 (9.7) | 0.56 | 0.52 | 70 | 68 | 2 | 98.1 | 92.6 | 5.5 |
| 11 | 14. | 4 (14.6) | 0.69 | 0.54 | 63 | 66 | -3 | 99.7 | 95.6 | 4.1 |
| 12 | 15. | 5 (9.7) | 0.42 | 0.32 | 77 | 80 | -3 | 95.9 | 93.3 | 2.6 |
| 13 | 16. | 5 (14.6) | 0.31 | 0.26 | 83 | 84 | -1 | 101.2 | 98.2 | 3.0 |
| | D. Y-2 Image Coupler | | | | | | | | | |
| - | 17. | None (control) | 2.76 | 1.51 | - | - | - | 88.6 | 86.3 | 2.0 |
| 14 | 18. | C-1 (9.7) | 1.45 | 0.86 | 47 | 343 | -4 | 94.2 | 90.8 | 3.4 |
| 15 | 19. | 4 (14.6) | 1.38 | 0.69 | 50 | 54 | -4 | 94.3 | 91.5 | 2.8 |
| 16 | 20. | 5 (9.7) | 1.15 | 0.58 | 58 | 362 | -4 | 95.6 | 94.0 | 1.6 |

*Value in parenthesis is the image modifying coupler level as a mole percent of image coupler; Y-1 coated at 1291 mg (1.44 mmol)/$m^2$; Y-2 at 1076 mg (1.34 mmol)/$m^2$.

**Sharpness as CMT for the causer (c) or receiver (r) layer on 1/60 sec. neutral exposure.

Since coupling rates and t1/2 values are each comparable among these couplers, the more negative C-R value may be interpreted as an enhanced tendency for the $T_1$-PMT fragment to diffuse farther before releasing inhibitor.

In contrast to the comparison couplers, the image modifying couplers have a first timing group ($T_1$) and a second timing group ($T_2$) in sequence. Attachment of hydrophilic groups such as -COOH in compounds 4 and 5 (see Tables 3C and D) provided more negative C-R values than for compounds 1 through 3, indicating a greater tendency for timely action on the receiver layer. However, these values varied over a smaller range, so that a balance of causer versus receiver effects closer to that of comparison coupler C-1 were achieved. It can be seen from Table 3 that image modifying couplers giving the greatest inhibition in a causer or receiver layer generally provided the greatest sharpness improvements in that layer. An unexpected 3 CMT improvement in receiver sharpness at comparable gamma was observed for sample 14 (invention Example 11) vs. sample 13 (comparison Example 10).

## Sharpness

When only the causer layer was exposed and developed, the released inhibitor acted predominantly in that layer and provided greater sharpness than when the inhibition effects were spread between two layers. The data in following Table 4 show that image modifying coupler 5 of the invention when compared with comparison coupler C-1 at the same molar concentration (sample 20 vs. 18) provided unexpected improvements of 1 to 3 in CMT acutance, increasing in the higher density steps. A 50% higher molar concentration of coupler 4 (sample 19) also gave excellent sharpness but the largest improvements appeared in the lower density steps. Again a general correlation of sharpness was observed with the extent of inhibition, here measured as % $\Delta$D.

Table 4

| Causer Layer Sharpness | | | | | |
|---|---|---|---|---|---|
| Example No. | Sample No. | D* | %ΔD | CMT-16 | CMT-35 |
| | A. Step 1 | | | | |
| - | 17. Control | 0.97 | 0 | 90.4 | 98.6 |
| 17 | 18. Comparison Coupler C-1 | 0.57 | 41 | 93.2 | 99.9 |
| 18 | 19. InventionCoupler 4 | 0.49 | 49 | 96.0 | 102.2 |
| 19 | 20. Invention Coupler 5 | 0.45 | 54 | 94.5 | 101.1 |
| | B. Step 2 | | | | |
| - | 17. Control | 1.66 | 0 | 90.2 | 98.7 |
| 20 | 18. Comparison Coupler C-1 | 1.00 | 40 | 97.0 | 102.0 |
| 21 | 19. Invention Coupler 4 | 0.88 | 47 | 97.0 | 103.4 |
| 22 | 20. Invention Coupler 5 | 0.79 | 52 | 98.6 | 103.9 |
| | C. Step 3 | | | | |
| - | 17. Control | 1.87 | 0 | - | - |
| 23 | 18. Comparison Coupler C-1 | 1.36 | 27 | 97.6 | 102.5 |
| 24 | 19. Invention Coupler 4 | 1.26 | 33 | 98.7 | 104.4 |
| 25 | 20. Invention Coupler 5 | 1.11 | 41 | 100.5 | 105.6 |

*D = Density - Dmin of causer layer
Steps 1, 2 and 3 represent exposures increasing by 0.3 logE increments on film samples described in Table 3D.

Examples of other compounds which can be prepared by methods as described and which are useful in photographic elements are as follows: (Examples 26 through 34 illustrate development inhibitor releasing couplers according to the invention.)

Example 26

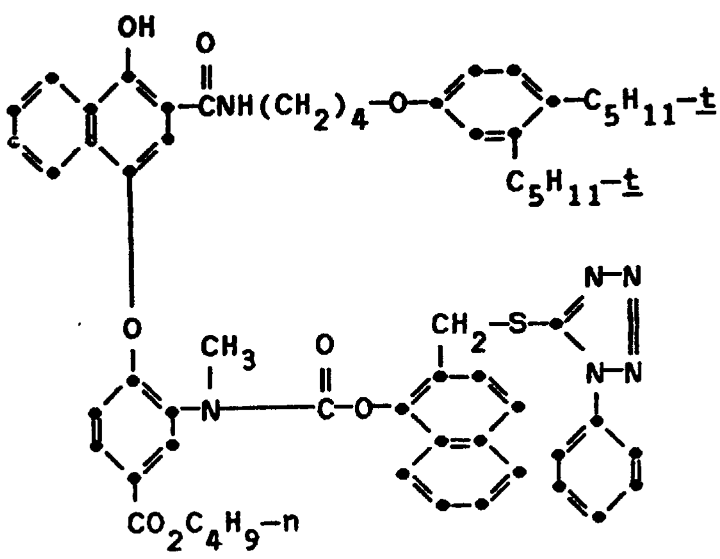

Example 27

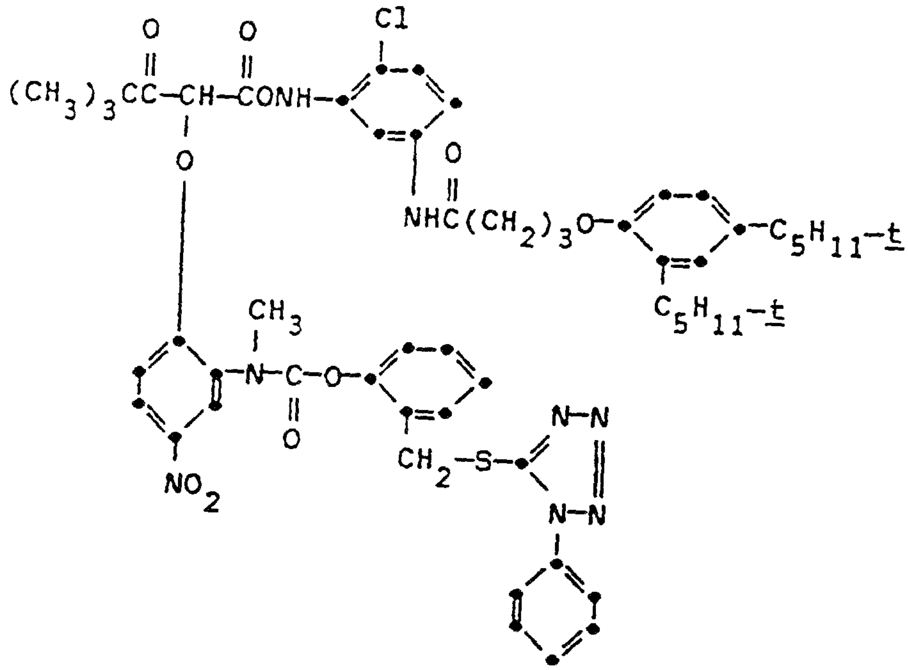

Example 28 (reference)

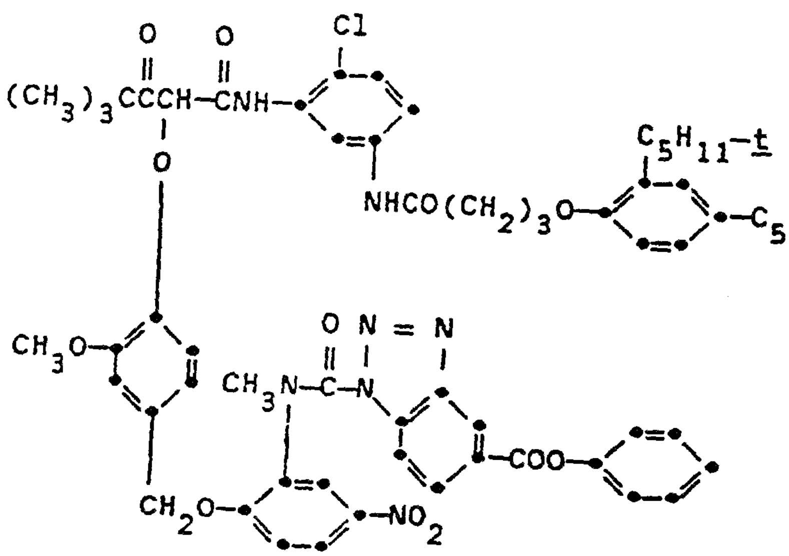

example 29 (reference)

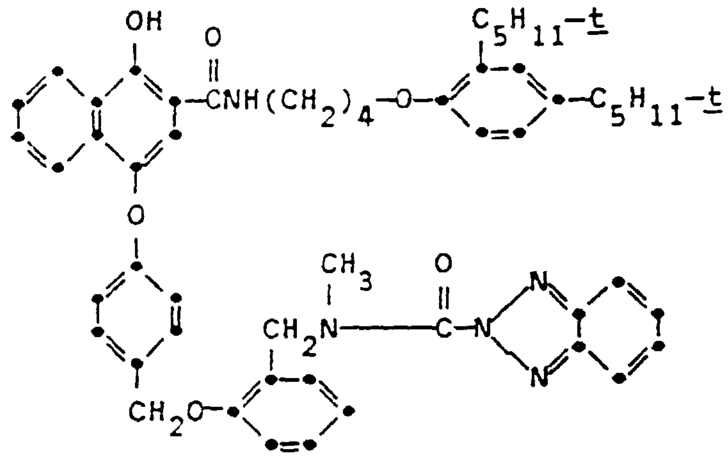

Example 30

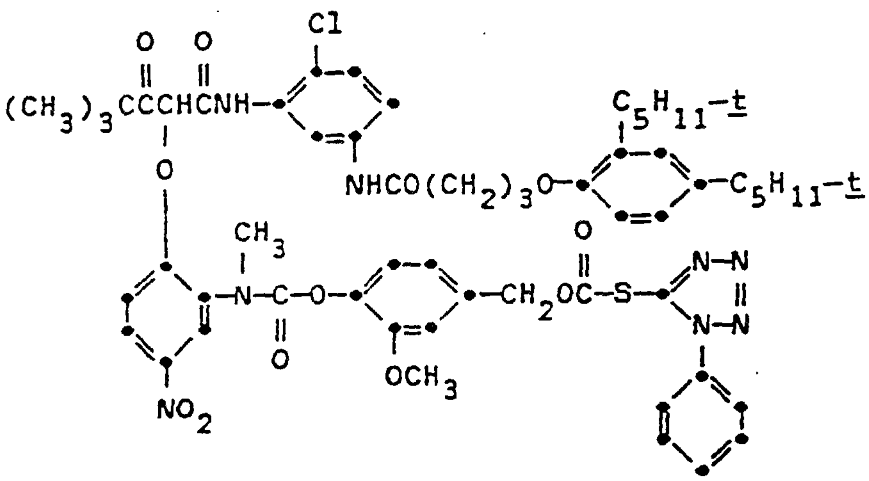

Example 31

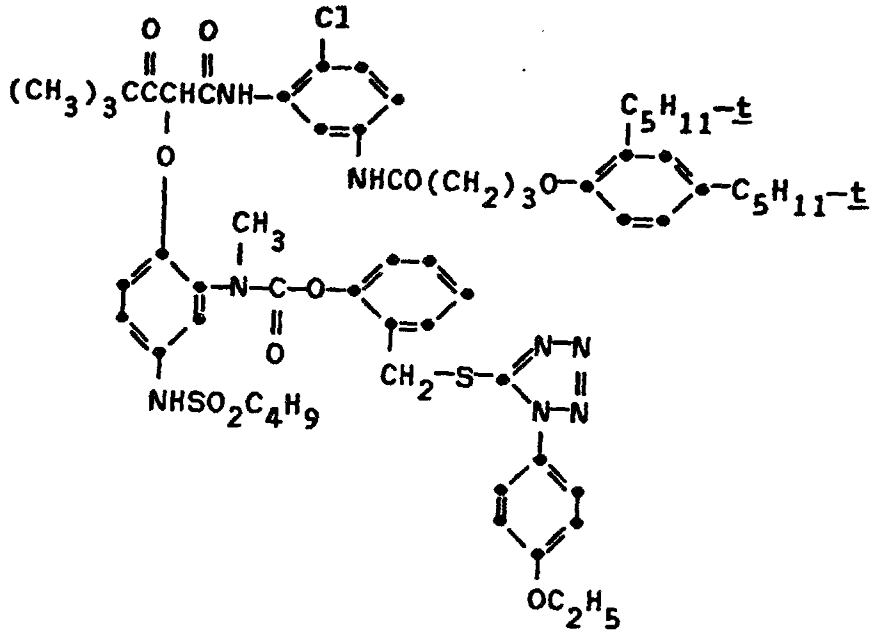

Example 32

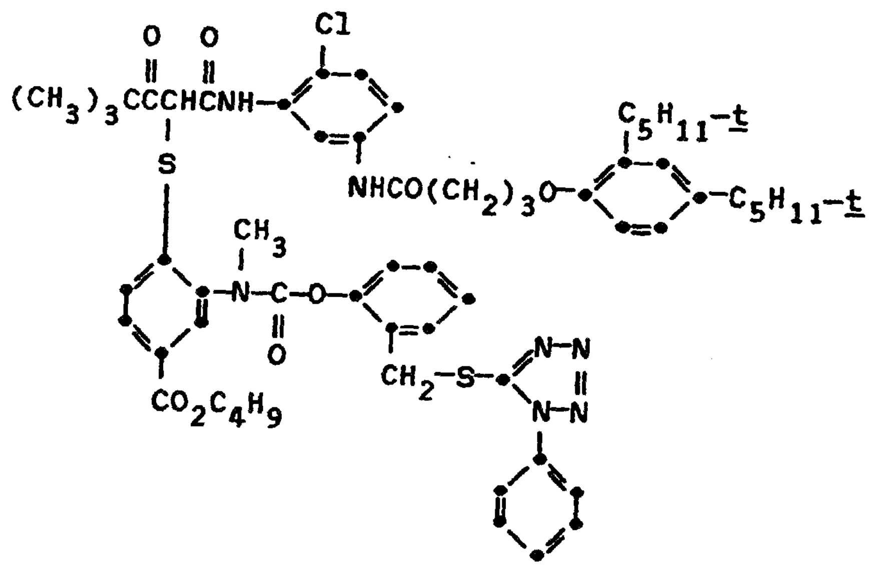

Example 33

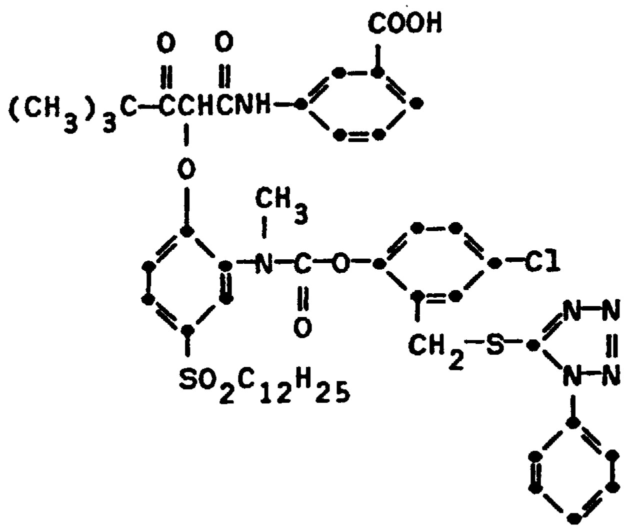

Example 34

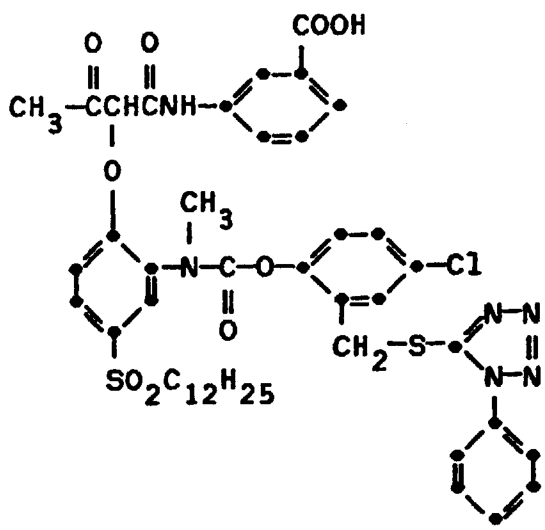

Example 35

This illustrates a development accelerator releasing coupler:

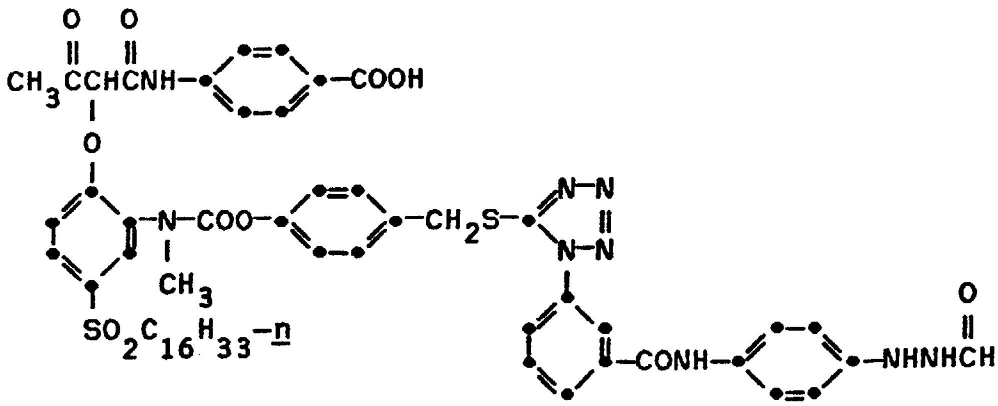

Example 36

This illustrates an image stabilizer releasing coupler:

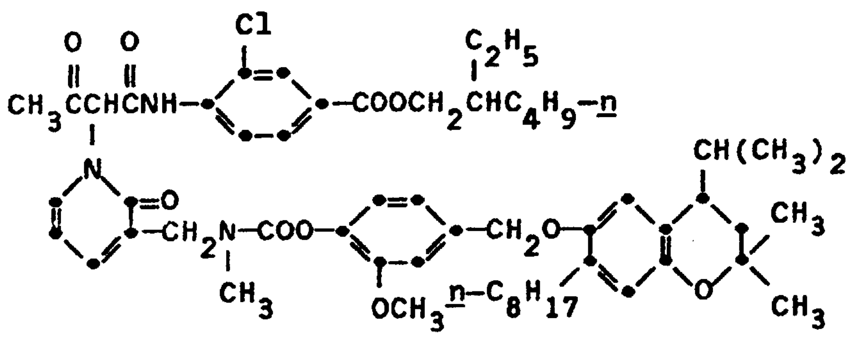

Example 37

This illustrates a bleach inhibitor releasing coupler:

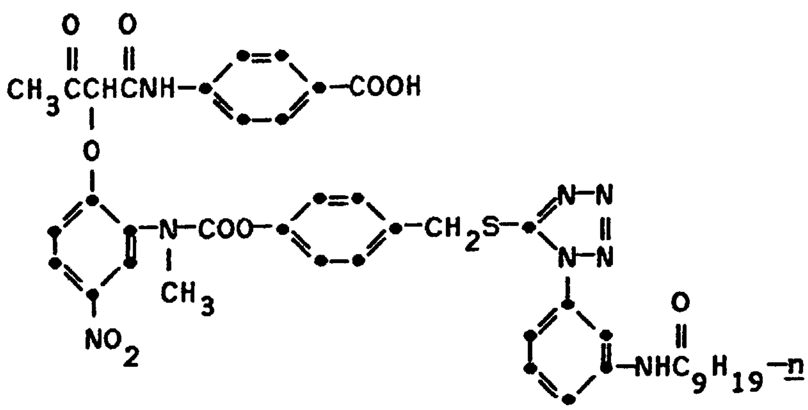

Example 38

This illustrates a bleach accelerator releasing coupler:

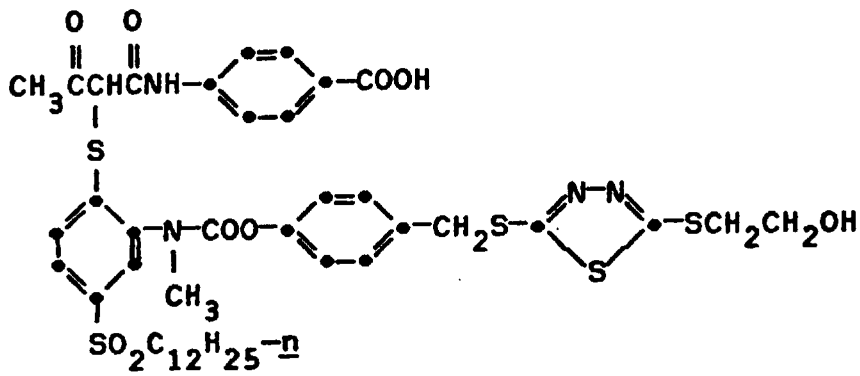

Example 39

This illustrates a nucleator releasing coupler:

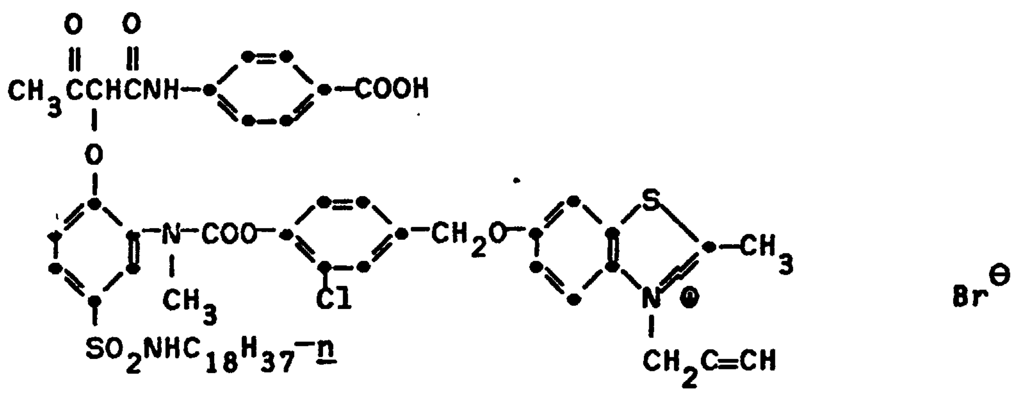

Example 40

This illustrates a competing coupler releasing coupler:

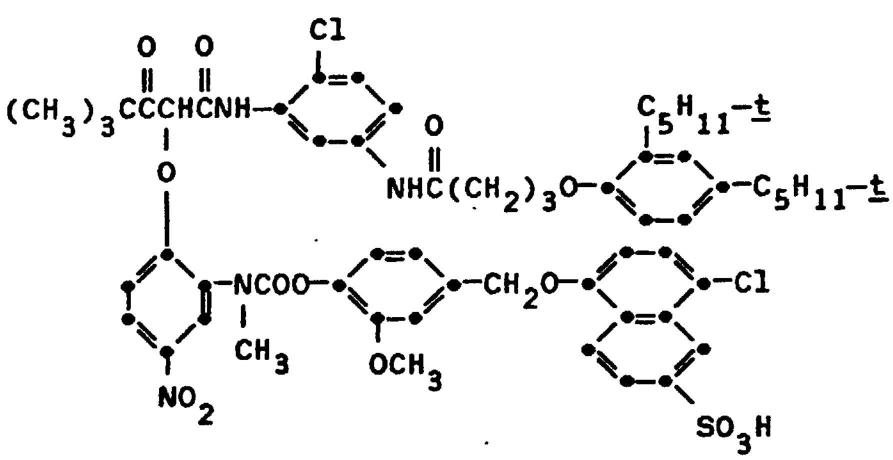

Example 41

This illustrates an image coupler releasing coupler:

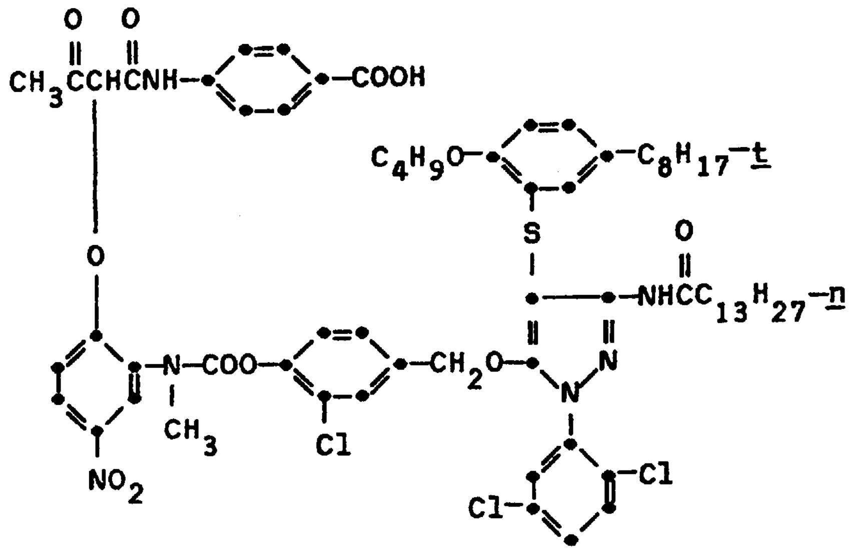

Example 42

This illustrates a developing agent releasing couper

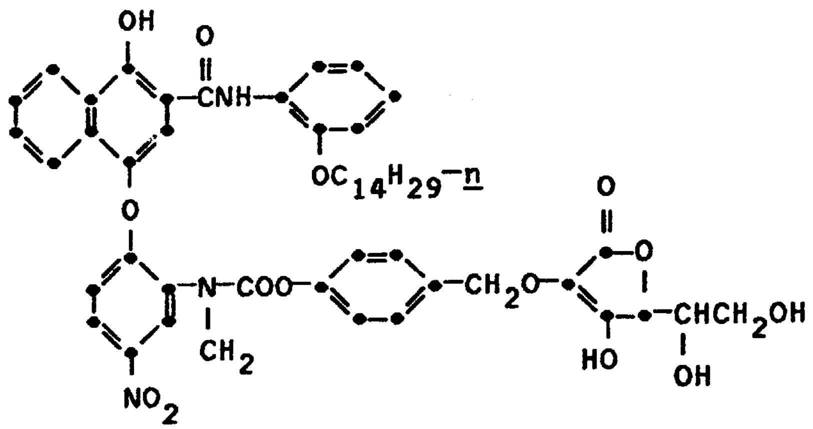

Example 43

This illustrates a fixing agent releasing coupler:

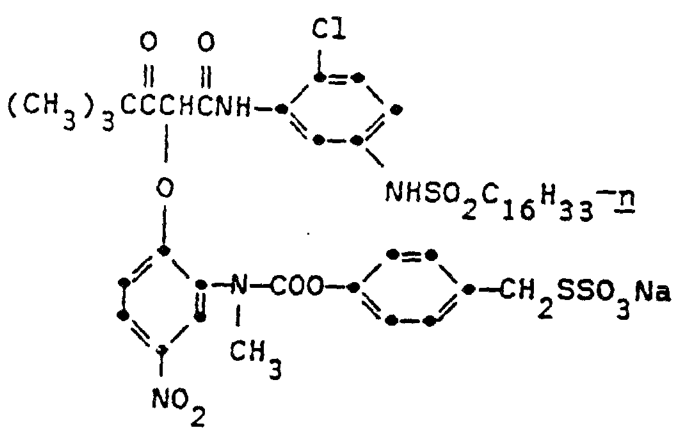

Example 44 (reference)

This illustrates a hardener releasing coupler:

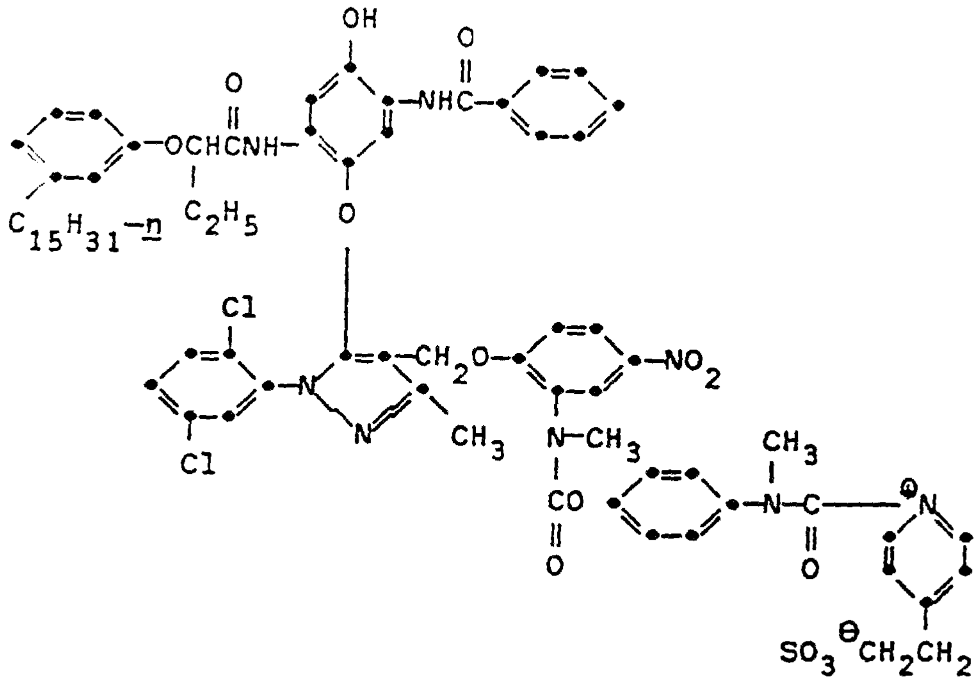

Example 45

This illustrates a toner releasing coupler:

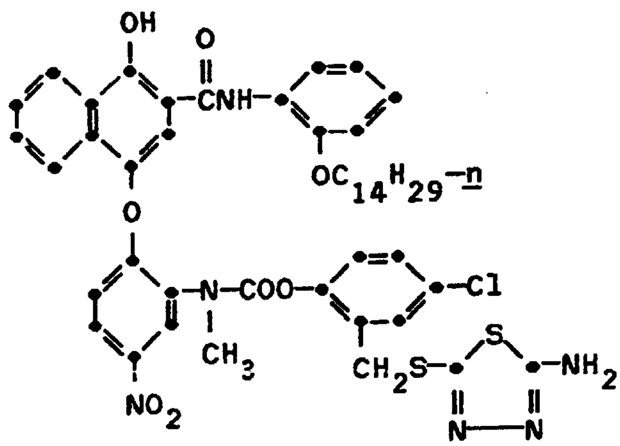

Example 46

This illustrates an antifoggant releasing coupler:

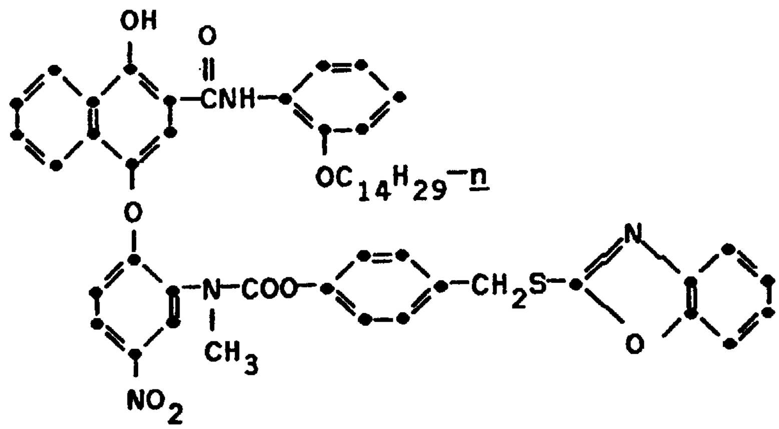

Example 47

This illustrates a dye releasing coupler:

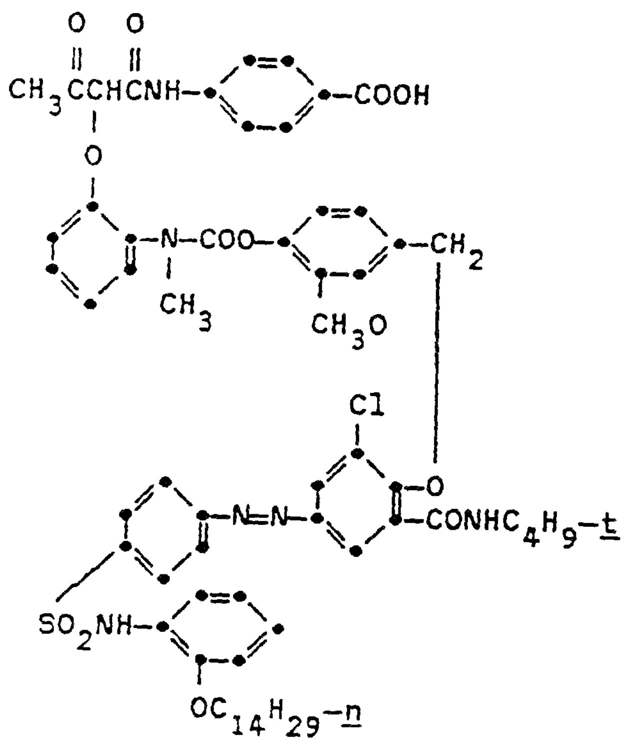

example 48

This illustrates a coupler which has a first timing group capable of releasing a development accelerator and a second timing group that is capable of releasing a development inhibitor:

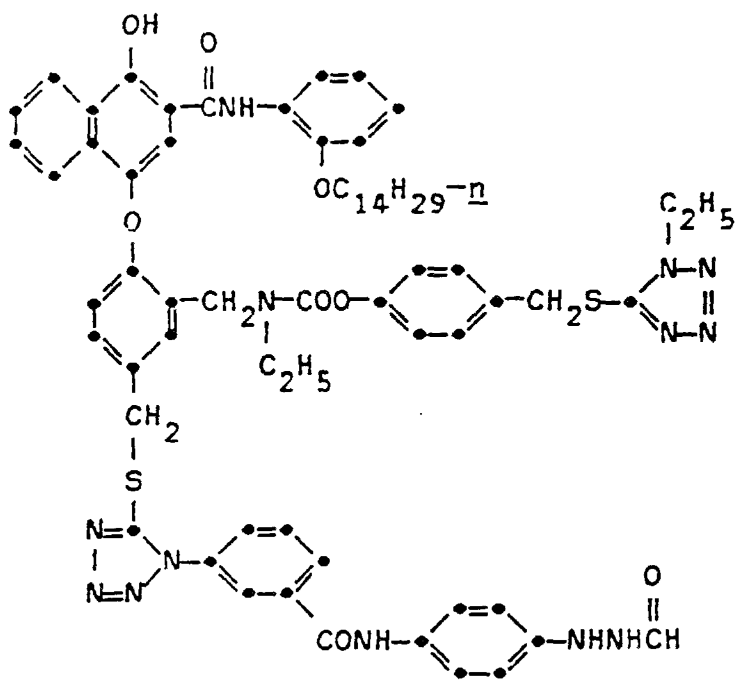

Example 49

This illustrates a polymeric coupler:

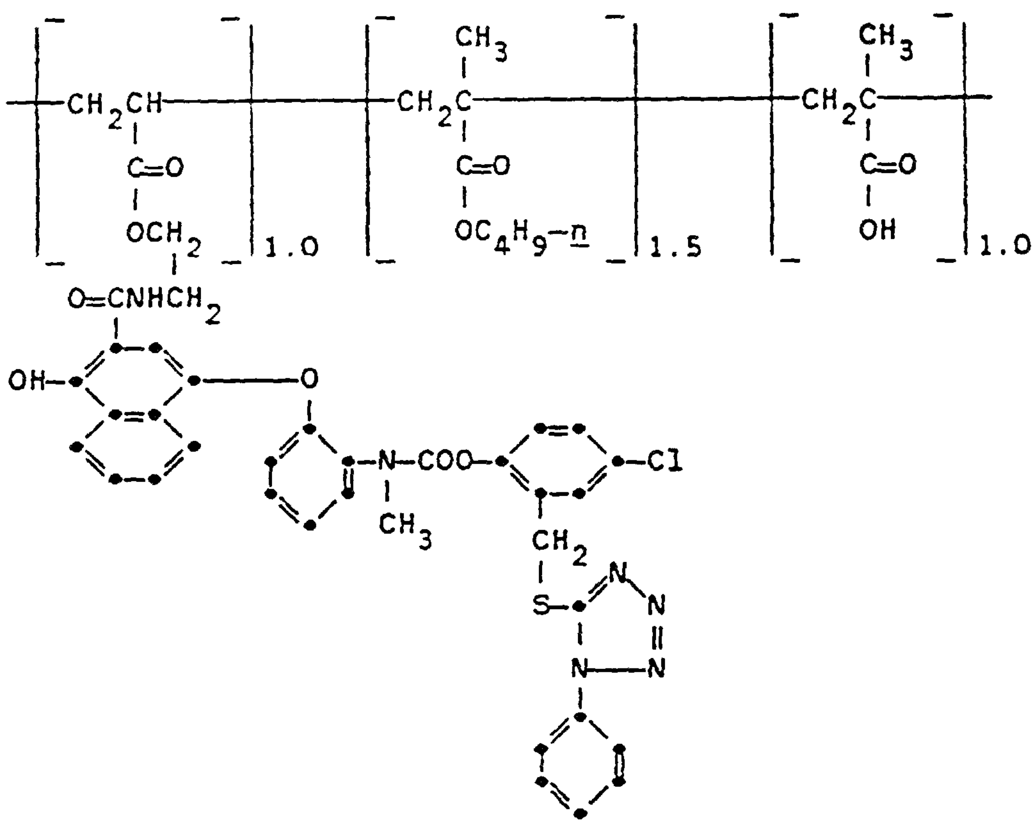

Another illustrative photographic element, as described, comprising a coupler represented by the formula:

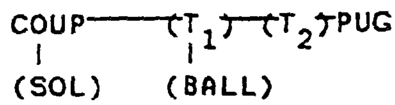

wherein

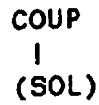

is a coupler moiety having a water solubilizing group (SOL) which enables the coupler moiety, upon release of the remainder of the coupler, to become a mobile moiety in the photographic element upon reaction with oxidized color developer;

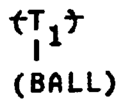

is a first timing group having a ballast group (BALL) substituent which enables the coupler before processing to be immobile and then enables the first timing group to be immobile after reaction of the coupler with oxidized color developer;

BALL is a ballast group which enables the coupler before processing to be immobile and then during and after processing enables the first timing group to be immobile;
SOL is a water solubilizing group which enables the coupler moiety to be mobile after reaction of the coupler with oxidized color developing agent;
$T_2$ is a second timing group, different from the first timing group $T_1$; and
PUG is a photographically useful group.

Upon exposure of the photographic element and processing, oxidized color developing agent reacts with the initially immobile coupler to form a mobile dye and an immobile first fragment

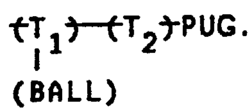

After a time delay, a second fragment which can be a mobile $\{T_2\}$ PUG is released from immobile

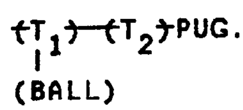

Finally after another time delay the PUG is released from $\{T_2\}$ PUG. PUG can be mobile or immobile. In one example, PUG is a development inhibitor. The coupler can serve, for example, as a development inhibitor releasing (DIR) coupler wherein the coupler upon processing leaves no residual dye in the imaging layer because the dye formed is water soluble and is removed from the imaging layer due to the water solubilizing group of the coupler moiety.

**Claims**

1. A photographic element comprising a support, at least one photographic emulsion layer and at least one compound A of the following formula:

$$COUP - (T_1) - (T_2)\text{-}PUG$$

wherein:

COUP is a coupler moiety;

PUG is a photographically useful group and

$T_1$ and $T_2$ are different first and second timing groups, which control the release of PUG, which is bonded to $T_2$; the first timing group $T_1$ is capable of being released from COUP at the coupling position; said compound A forms two different fragments from the two different timing groups $T_1$ and $T_2$ upon processing the photographic element, wherein

$T_1$ is a group capable of an intramolecular nucleophilic displacement reaction, said group comprising a nucleophilic and an electrophilic group which form a cyclic organic ring or a transient cyclic organic ring by the intramolecular reaction, and

$T_2$ is a group capable of electron transfer down a conjugated chain.

**2.** A photographic element according to claim 1 wherein the compound A is:

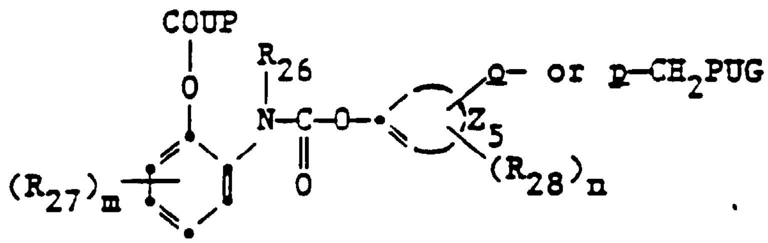

wherein:

$R_{26}$ is substituted or unsubstituted alkyl or substituted or unsubstituted aryl;
$R_{27}$ is hydrogen or a substituent which does not adversely affect timing of release of the other portions of the compound A;
$R_{28}$ is hydrogen or a substituent which advantageously influences the timing of release of PUG;
COUP is a coupler moiety substituted in the coupling position by the remainder of the coupler;
PUG is a photographically useful group;
m and n are individually 0 or 1;
$Z_5$ represents the atoms necessary to complete a carbocyclic or heterocyclic ring.

**3.** A photographic element as in any of claims 1 or 2 wherein the compound A is:

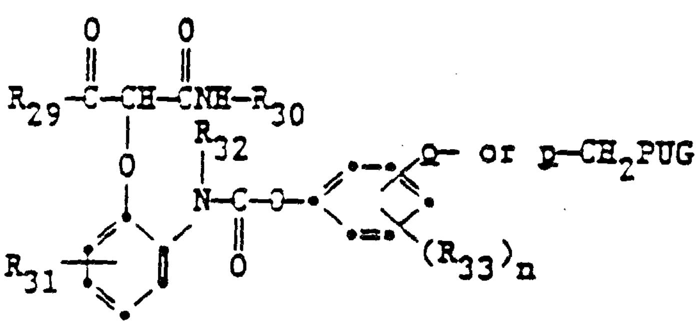

wherein:

$R_{29}$ is substituted or unsubstituted alkyl, substituted or unsubstituted aryl, or $R_{34}NH$;
$R_{30}$ is substituted or unsubstituted alkyl, or substituted or unsubstituted aryl;
$R_{31}$ is a hydrogen or a ballast group;
$R_{32}$ is alkyl;
$R_{33}$ is unsubstituted or substituted alkyl, chlorine, bromine, nitro, or alkoxy;
$R_{34}$ is substituted or unsubstituted alkyl, or substituted or unsubstituted aryl;
PUG is a photographically useful group; and
n is 0, 1 or 2.

**4.** A photographic element as in any of claims 1 - 4 wherein the compound A is:

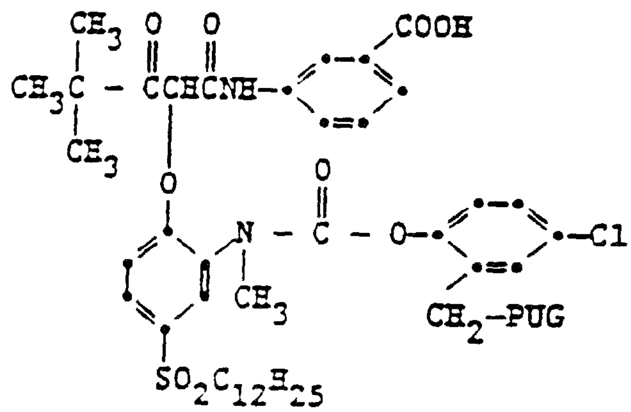

wherein PUG is a photographically useful group.

5. A process of forming a photographic image which comprises developing an exposed photographic silver halide emulsion layer with a color developing agent in the presence of a compound A as defined in any of claims 1 - 4 .

6. A compound A comprising a coupler moiety and bonded thereto, in sequence, a first timing group, a second timing group different from the first timing group, and bonded to the second timing group, a photographically useful group; the compound A characterized in that its timing groups are defined as in any of claims 1 through 4.

## Patentansprüche

1. Photographisches Element mit einem Träger, mindestens einer photographischen Emulsionsschicht und mindestens einer Verbindung A der folgenden Formel:

$$COUP - (T_1) - (T_2)\text{-}PUG$$

in der bedeuten:

COUP einen Kupplerrest;

PUG eine photographisch nutzbare Gruppe, und

$T_1$ und $T_2$ verschiedene erste und zweite Steuergruppen, die die Freisetzung von PUG, das an $T_2$ gebunden ist, steuern; wobei die erste Steuergruppe $T_1$ von COUP an der Kupplungsposition freigesetzt werden kann; wobei die Verbindung A zwei verschiedene Fragmente von den zwei verschiedenen Steuergruppen $T_1$ und $T_2$ bei der Entwicklung des photographischen Elementes bildet, wobei

$T_1$ eine Gruppe ist, die einer intramolekularen nukleophilen Verdrängungsreaktion zugänglich ist, wobei die Gruppe eine nukleophile und eine elektrophile Gruppe umfaßt, die durch die intramolekulare Reaktion einen cyclischen organischen Ring oder einen cyclischen organischen Übergangsring bilden, und

$T_2$ eine Gruppe, die zu einem Elektronenübergang längs einer konjugierten Kette befähigt ist.

2. Photographisches Element nach Anspruch 1, in dem die Verbindung A die folgende Formel hat:

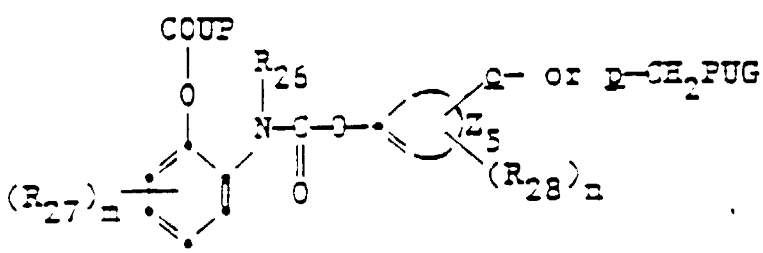

worin bedeuten:

$R_{26}$ substituiertes oder unsubstituiertes Alkyl oder substituiertes oder unsubstituiertes Aryl;

$R_{27}$ Wasserstoff oder einen Substituenten, der die Steuerung der Freisetzung der anderen Teile der Verbindung A nicht nachteilig beeinflußt;

$R_{28}$ Wasserstoff oder einen Substituenten, der in vorteilhafter Weise die Steuerung der Freisetzung von PUG beeinflußt;

COUP einen Kupplerrest, der in der Kupplungsposition durch den Rest des Kupplers substituiert ist;

PUG eine photographisch nutzbare Gruppe;

m und n einzeln 0 oder 1;

$Z_5$ die Atome, die zur Vervollständigung eines carbocyclischen oder heterocyclischen Ringes erforderlich sind.

**3.** Photographisches Element nach einem der Ansprüche 1 oder 2, worin die Verbindung A die folgende Formel hat:

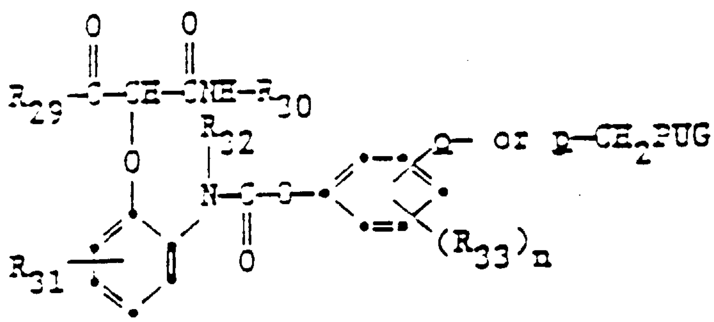

worin bedeuten:

$R_{29}$ substituiertes oder unsubstituiertes Alkyl, substituiertes oder unsubstituiertes Aryl oder $R_{34}$NH;

$R_{30}$ substituiertes oder unsubstituiertes Alkyl oder substituiertes oder unsubstituiertes Aryl;

$R_{31}$ Wasserstoff oder eine Ballastgruppe;

$R_{32}$ Alkyl;

$R_{33}$ unsubstituiertes oder substituiertes Alkyl, Chlor, Brom, Nitro oder Alkoxy;

$R_{34}$ substituiertes oder unsubstituiertes Alkyl oder substituiertes oder unsubstituiertes Aryl;

PUG eine photographisch nutzbare Gruppe; und

n gleich 0, 1 oder 2.

**4.** Photographisches Element nach einem der Ansprüche 1 - 4, worin die Verbindung A die folgende Formel hat:

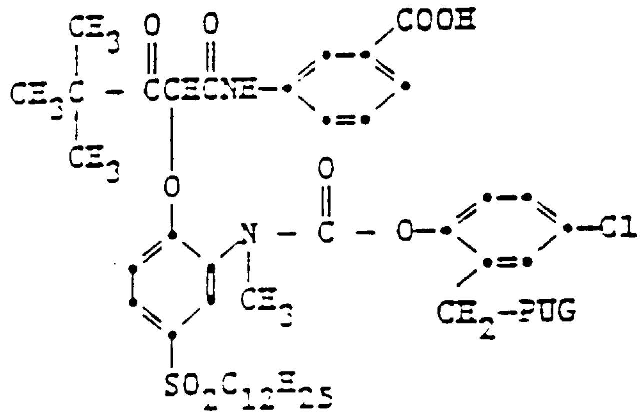

worin PUG eine photographisch nutzbare Gruppe ist.

**5.** Verfahren zur Herstellung eines photographischen Bildes, das die Entwicklung einer exponierten photographischen Silberhalogenidemulsionsschicht mit einer Farbentwicklerverbindung in Gegenwart einer Verbindung A wie in einem der Ansprüche 1 - 4 definiert, umfaßt.

**6.** Eine Verbindung A mit einem Kupplerrest und hieran in Folge gebunden einer ersten Steuergruppe, einer zweiten Steuergruppe, die von der ersten Steuergruppe verschieden ist und mit einer an die zweite Steuergruppe gebundenen photographisch nutzbaren Gruppe; wobei die Verbindung A dadurch gekennzeichnet ist, daß die Steuergruppen wie in einem der Ansprüche 1 - 4 angegeben definiert sind.

### Revendications

**1.** Elément photographique comprenant un support, au moins une couche d'émulsion photographique et au moins un composé A de formule :

$$COUP - (T_1) - (T_2) - PUG$$

où :

COUP est un groupement coupleur ;
PUG est un groupe photographiquement utile, et
$T_1$ et $T_2$ sont un premier et un second groupes retardateurs différents contrôlant la libération de PUG qui est rattaché à $T_2$; le premier groupe retardateur $T_1$ est capable d'être libéré de COUP en position de couplage ; ledit composé A forme deux fragments différents à partir des deux groupes retardateurs différents $T_1$ et $T_2$ au cours du développement de l'élément photographique, où :
$T_1$ est un groupe capable d'une réaction de déplacement nucléophile intramoléculaire, ledit groupe comprenant un groupe nucléophile et un groupe électrophile formant un cycle organique ou un cycle organique transitoire au moyen de la réaction intramoléculaire, et $T_2$ est un groupe capable de transfert électronique le long d'une chaîne conjuguée.

**2.** Elément photographique selon la revendication 1, dans lequel le composé A est :

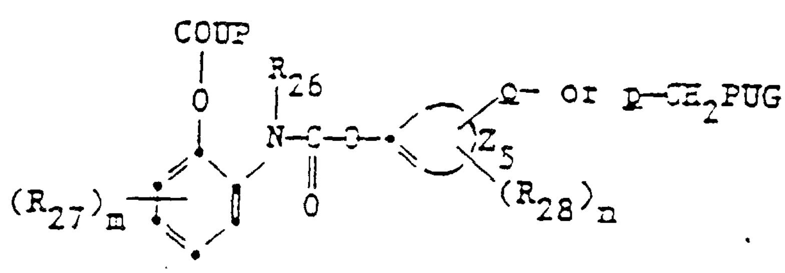

où :

$R_{26}$ est un groupe alkyle ou aryle substitué ou non ;
$R_{27}$ est l'hydrogène ou un substituant n'influençant pas défavorablement la libération en temps voulu des autres parties du composé A ;
$R_{28}$ est l'hydrogène ou un substituant favorisant la libération en temps voulu de PUG ;
COUP est un groupement coupleur substitué en position de couplage par le reste du coupleur ;
PUG est un groupe photographiquement utile ; m et n sont individuellement 0 ou 1 ;
$Z_5$ représente les atomes nécessaires pour compléter un hétérocycle ou un carbocycle.

**3.** Elément photographique selon l'une quelconque des revendications 1 ou 2, dans lequel le composé A est :

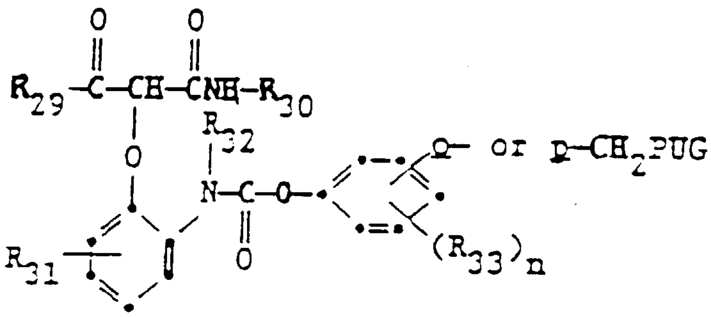

où :

$R_{29}$ est un groupe alkyle ou aryle substitué ou non, ou $R_{34}NH$ ;
$R_{30}$ est un groupe alkyle ou aryle substitué ou non ;
$R_{31}$ est l'hydrogène ou un groupe ballast ;
$R_{32}$ est un groupe alkyle ;
$R_{33}$ est un groupe alkyle substitué ou non, chlore, brome, nitro ou alcoxy ;
$R_{34}$ est un groupe alkyle ou aryle substitué ou non ;
PUG est un groupe photographiquement utile ; et
n est 0, 1 ou 2.

**4.** Elément photographique selon l'une quelconque des revendications 1 - 4, dans lequel le composé A est :

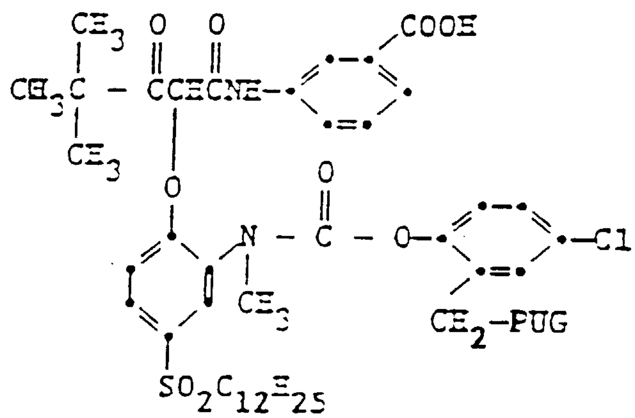

où :
PUG est un groupe photographiquement utile.

**5.** Procédé de formation d'une image photographique consistant à développer une couche d'émulsion photographique aux halogénures d'argent avec un développateur chromogène en présence d'un composé A, tel que défini dans l'une quelconque des revendications 1 - 4.

**6.** Composé A comprenant un groupement coupleur et rattaché à celui-ci, dans l'ordre, un premier groupe retardateur,

un second groupe retardateur différent du premier groupe retardateur, et rattaché à ce second groupe retardateur, un groupe photographiquement utile ; le composé A étant caractérisé en ce que ses groupes retardateurs sont définis dans l'une quelconque des revendications 1 à 4.